# EUROPÄISCHE PATENTSCHRIFT

(11) **EP 4 076 366 B1**
(45) Veröffentlichungstag und Bekanntmachung des Hinweises auf die Patenterteilung: **12.08.2026**
(21) Anmeldenummer: 20793627.9
(22) Anmeldetag: 16.10.2020
(51) Int. Cl.: A61K 8/46, A61K 8/58, A61K 8/81, A61Q 5/06

(54) **VERFAHREN ZUM FÄRBEN VON KERATINISCHEM MATERIAL, UMFASSEND DIE ANWENDUNG VON EINER SILICIUMORGANISCHEN VERBINDUNG, EINER FARBGEBENDEN VERBINDUNG UND EINES VORBEHANDLUNGSMITTELS**
METHOD FOR DYEING KERATINOUS MATERIAL, COMPRISING THE USE OF AN ORGANOSILICON COMPOUND, A DYEING COMPOUND AND A PRE-TREATMENT AGENT
PROCÉDÉ DE TEINTURE DE MATÉRIAU KÉRATINIQUE, COMPRENANT L'UTILISATION D'UN COMPOSÉ D'ORGANOSILICIUM, UN COMPOSÉ DE TEINTURE ET UN AGENT DE PRÉTRAITEMENT

(30) Priorität: 16.12.2019 DE 102019219718
(43) Veröffentlichungstag der Anmeldung: 26.10.2022
(73) Patentinhaber: Henkel AG & Co. KGaA, 40589 Düsseldorf (DE)
(72) Erfinder: KROHN, Rene, 22848 Norderstedt (DE); HIPPE, Thomas, 25482 Appen (DE); BRENDER, Jessica, 22763 Hamburg (DE); HOEPFNER, Stefan, 22523 Hamburg (DE)
(86) Internationale Anmeldenummer: PCT/EP2020/079181
(87) Internationale Veröffentlichungsnummer: WO 2021/121721

(56) Entgegenhaltungen:
- EP-A2- 2 168 633
- MARIA FERNANDAREIS GAVAZZONI DIAS: "Hair cosmetics: An overview", INTERNATIONAL JOURNAL OF TRICHOLOGY, vol. 7, no. 1, 1 January 2015 (2015-01-01), pages 2, XP055757932, ISSN: 0974-7753, DOI: 10.4103/0974-7753.153450

## Beschreibung

Gegenstand der vorliegenden Anmeldung ist ein Verfahren zum Behandeln von keratinischem Material, insbesondere menschlichen Haaren, welches die Anwendung von zwei Mitteln (a) und (b) sowie eines Vorbehandlungsmittels umfasst. Das Mittel (a) ist durch seinen Gehalt an mindestens einer organischen Siliciumverbindung (a1) gekennzeichnet. Das Mittel (b) enthält mindestens ein Versiegelungsreagenz (b1). Ferner enthält entweder das Mittel (a) oder das Mittel (b) oder es enthalten beide Mittel (a) und (b) mindestens eine farbgebende Verbindung aus der Gruppe der Pigmente und/oder der direktziehenden Farbstoffe.

Ein weiterer Gegenstand dieser Anmeldung ist eine Mehrkomponenten-Verpackungseinheit (Kit-ofparts) zum Färben von keratinischem Material, insbesondere menschlichen Haaren, welche getrennt voneinander konfektioniert mindestens drei Mittel (a'), (a") und (b) sowie ein Vorbehandlungsmittel umfasst. Aus den Mitteln (a') und (a") kann das im oben beschriebenen Verfahren angewendete Mittel (a) hergestellt werden.

Noch ein weiterer Gegenstand dieser Anmeldung ist eine Mehrkomponenten-Verpackungseinheit (Kitof-parts) zum Färben von keratinischem Material, insbesondere menschlichen Haaren, welche getrennt voneinander konfektioniert mindestens vier Mittel (a'), (a"), (a‴) und (b) sowie ein Vorbehandlungsmittel umfasst. Aus den Mitteln (a'), (a") und (a‴) kann das im oben beschriebenen Verfahren angewendete Mittel (a) hergestellt werden.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit Tensid-haltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung der Kombination aus einem Pigment, einer organischer Siliciumverbindung, einem filmbildenden Polymer und einem Lösungsmittel auf Haaren Färbungen erzeugen lassen, die gegenüber Abrieb und/oder Shampoonierungen besonders widerstandsfähig sind.

Es besteht Bedarf, Haarfärbungen mit Pigmenten bereitzustellen, die einerseits eine hohe Wasch- und Reibechtheit und andererseits die Haareigenschaften wie Handhabbarkeit und Haptik nicht negativ beeinträchtigen. Dazu wäre es wünschenswert, intensive Färbungen durch einen guten und gleichmäßigen Aufzug der Pigmente auf dem keratinischen Material zu erhalten.

Entsprechend war die Aufgabe der vorliegenden Erfindung, ein Färbesystem mit Pigmenten bereitzustellen, das mit der oxidativen Färbung vergleichbare Echtheitseigenschaften besitzt. Insbesondere die Waschechtheiten sollten herausragend sein, hierbei sollte jedoch auf den Einsatz der sonst zu diesem Zweck üblicherweise eingesetzten Oxidationsfarbstoffvorprodukte verzichtet werden. Insbesondere sollte auch eine gleichmäßige und lang anhaltende Färbung erreicht werden.

Überraschenderweise hat sich nun herausgestellt, dass die vorgenannte Aufgabe hervorragend gelöst werden kann, wenn keratinische Materialien, insbesondere menschliche Haare, mit einem Verfahren gefärbt werden, bei welchem zunächst ein Vorbehandlungsmittel und anschließend mindestens zwei Mittel (a) und (b) auf die keratinischen Materialien (Haare) appliziert werden. Hierbei enthält das erste Mittel (a) mindestens eine organische Siliciumverbindung aus der Gruppe der Silane mit einem, zwei oder drei Siliciumatomen. Das zweite Mittel (b) enthält mindestens ein Versiegelungsreagenz. Ferner enthält entweder das Mittel (a) oder das Mittel (b) oder es enthalten beide Mittel (a) und (b) mindestens eine farbgebende Verbindung aus der Gruppe der Pigmente und/oder der direktziehenden Farbstoffe.

Bei Einsatz der zwei Mittel (a) und (b) in einem Färbeverfahren konnte keratinisches Material in besonders hoher Farbintensität und hohen Echtheiten gefärbt werden.

Durch eine Vorbehandlung des keratinischen Materials mit einem Vorbehandlungsmittel konnte ein überraschend gleichmäßiger und langanhaltender Aufzug der mindestens einen farbgebende Verbindung erreicht werden.

Ein erster Gegenstand der vorliegenden Erfindung ist ein Verfahren zum Färben von keratinischem Material, insbesondere menschlichen Haaren, umfassend die folgenden Schritte:
- in einem ersten Schritt Anwendung eines Mittels (v) mit einen pH-Wert im Bereich von 9,8 bis 10,2 auf dem keratinischem Material, wobei das Mittel (v) enthält
   (v1) mindestens ein anionisches Tensid,
- in einem zweiten Schritt Anwendung eines Mittels (a) auf dem keratinischem Material, wobei das Mittel (a) enthält
   (a1) mindestens eine organische Siliciumverbindung aus der Gruppe der Silane mit einem, zwei oder drei Siliciumatomen, und
- in einem dritten Schritt Anwendung eines Mittels (b) auf dem keratinischen Material, wobei das Mittel (b) enthält
   (b1) mindestens ein Versiegelungsreagenz, umfassend eine Verbindung ausgewählt aus der Gruppe bestehend aus filmbildenden Polymeren, Alkalisierungsmitteln, Acidifizierungsmitteln und Mischungen daraus,
wobei mindestens eines der Mittel (a) und (b) ferner mindestens eine farbgebende Verbindung aus der Gruppe der Pigmente und/oder der direktziehenden Farbstoffe enthält.

Bei den zu dieser Erfindung führenden Arbeiten hat sich herausgestellt, dass die bevorzugt sukzessive Anwendung der Mittel (a) und (b) die Erzeugung von sehr stabilen und waschechten Färbungen auf den keratinischen Materialien ermöglicht. Die Anwendung der organischen Siliciumverbindung (a1) führt zur Ausbildung eines besonders resistenten Films auf dem keratinischen Material. Mit Applikation des zweiten Mittels (b) wird der auf dem keratinischen Material aufgebrachte Film versiegelt und somit widerstandsfähiger gegen Waschen und/oder Abrieb gemacht. Durch

Einsatz mindestens einer farbgebenden Verbindung aus der Gruppe der Pigmente und/oder der direktziehenden Farbstoffe in mindestens eines der Mittel (a) und (b) können gefärbte Filme erhalten werden.

Die farbgebenden Verbindungen können auf diesem Wege dauerhaft auf dem keratinischen Material fixiert werden, so dass überaus waschechte Färbungen mit guter Resistenz gegenüber Abrieb und/oder Shampoonierungen erhalten werden konnten.

Mit Hilfe des Vorbehandlungsmittels konnte der Aufzug der farbgebenden Verbindungen in den erzeugten Filmen deutlich erhöht werden und homogener erfolgen. Es wird vermutet, dass das Vorbehandlungsmittel die Oberfläche des keratinischen Materials egalisiert und beispielsweise vorhandene Produktrückstände (zum Beispiel Rückstände von angewendeten Stylingprodukten) und/oder substantive Verbindungen (zum Beispiel konditionierende Polymere aus angewendeten Konditioniermitteln), die einen gleichmäßigen und langanhaltenden Aufzug des durch die Anwendung der organischen Siliciumverbindung (a1) ausgebildeten Films verhindern, entfernt.

### Keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

### Vorbehandlungsmittel (v)

Im Rahmen des beschriebenen Verfahrens wird zunächst ein Vorbehandlungsmittel (v) auf dem keratinischen Material, insbesondere den menschlichen Haaren, appliziert. Das Vorbehandlungsmittel (v) ist gekennzeichnet durch einen Gehalt an mindestens einem anionischen Tensid (v1).

Gemäß einer bevorzugten Ausführungsform des Verfahrens sind die anionischen Tenside (v1) in dem Mittel (v) ausgewählt aus der Gruppe bestehend aus:
- geradkettigen oder verzweigten, gesättigten oder ein- oder mehrfach ungesättigten Alkylsulfonaten mit 8 bis 24, bevorzugt 12 bis 22, bevorzugter 16 bis 18 C-Atomen,
- linearen Alpha-Olefinsulfonaten mit 8 bis 24, bevorzugt 12 bis 22, bevorzugter 14 bis 18 C-Atomen,
- Alkylsulfaten und Alkylpolyglykolethersulfaten der Formel R₉-O-(CH₂-CH₂O)ₙ-SO₃X, in der R₉ bevorzugt für einen geradkettigen oder verzweigten, gesättigten oder ein- oder mehrfach ungesättigten Alkyl- oder Alkenlyrest mit 8 bis 24, bevorzugt 12 bis 22, bevorzugter 16 bis 18 Kohlenstoffatomen, n für 0 oder 1 bis 12, bevorzugter 2 bis 4 und X für ein Alkali- oder Erdalkalimetallion oder für protoniertes Triethanolamin oder das Ammonium-Ion steht,
- geradkettigen oder verzweigten, gesättigten oder ein- oder mehrfach ungesättigten Alkylcarbonsäuren mit 8 bis 24, bevorzugt 12 bis 22, bevorzugter 16 bis 18 C-Atomen,
- geradkettigen oder verzweigten, gesättigten oder ein- oder mehrfach ungesättigten Alkylphosphaten mit 8 bis 24, bevorzugt 12 bis 22, bevorzugter 16 bis 18 C-Atomen,
- Acylisethionaten, deren Acylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe
- Alkylglycosidcarbonsäuren, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Alkylsulfosuccinaten, dessen zwei Alkylgruppen ausgewählt sind aus gleichen oder verschiedenen, verzweigte oder unverzweigten C₂ bis C₁₂, bevorzugt C₄ bis C₁₀, bevorzugter C₆ bis C₈ Alkylgruppen,
- N-Acyltauraten, dessen Acylgruppe ausgewählt ist aus einer verzweigten oder unverzweigten C₆ bis C₂₂, bevorzugt C₈ bis C₁₈, bevorzugter C₁₂ bis C₁₈ Alkylgruppe,
- Alkylsarcosinaten, dessen Alkylgruppe ausgewählt ist aus einer verzweigten oder unverzweigten C₆ bis C₂₂, bevorzugt C₁₀ bis C₁₈, bevorzugter C₁₂ bis C₁₆ Alkylgruppe,
- Sulfonate ungesättigter Fettsäuren mit 8 bis 24, bevorzugt 12 bis 22, bevorzugter 16 bis 18 C-Atomen und 1 bis 6 Doppelbindungen und
- Mischungen daraus
wobei das/die Gegenionen des anionischen Tensids Alkali- oder Erdalkalimetallionen, protoniertes Triethanolamin oder das Ammonium-Ion umfasst/umfassen.

Besonders bevorzugte anionische Tenside (v1) sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten. Ganz besonders bevorzugt umfasst das mindestens eine anionische Tensid (v1) Natriumlaurylethersulfat (INCI: Sodium Laureth Sulfate) und ganz besonders bevorzugt Natriumlaurylethersulfat mit 2 Ethylenoxideinheiten.

Weiterhin besonders bevorzugte anionische Tenside (v1) sind geradkettige oder verzweigte Alkylsulfate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- Cetearyl- und/oder Myristylsulfate. Ganz besonders bevorzugt ist das Natriumsalz von Laurylsulfat (INCI: Sodium Lauryl Sulfate)

Ein bevorzugtes anionische Tenside (v1) aus der Gruppe der linearen Alpha-Olefinsulfonate ist insbesondere Natrium-α-Olefinsulfonat (INCI: Sodium C14-16 Olefin Sulfonate).

Bevorzugte anionische Tenside (v1) aus der Gruppe der Acylisethionate umfassen Verbindungen mit den INCI-Bezeichnungen Sodium Lauroyl Taurate, Sodium Methyl Cocoyl Taurate und Sodium Methyl Lauroyl Taurate.

Ein bevorzugtes anionische Tenside (v1) aus der Gruppe der Acylisethionate ist insbesondere Natriumcocoylisethionat.

Ein bevorzugtes anionische Tenside (v1) aus der Gruppe der Alkylphosphate ist insbesondere Sodium Lauryl Phosphate (INCI).

Ein bevorzugtes anionische Tenside (v1) aus der Gruppe der Alkylsulfosuccinate ist insbesondere Disodium Lauryl Sulfosuccinate (INCI).

Zur Erzielung eines besonders gleichmäßigen Färbeergebnisses hat es sich als bevorzugt herausgestellt, wenn das in dem Verfahren eingesetzte Vorbehandlungsmittel (v) ein anionisches Tensid aus der Gruppe der C₈₋₁₈-Alkylsulfatsalze, insbesondere das Natriumsalz von Laurylsulfat (INCI: Sodium Lauryl Sulfate), umfasst.

Entsprechend ist ein Verfahren bevorzugt bei dem das Vorbehandlungsmittel (v) enthält:
(v1) ein anionisches Tensid aus der Gruppe der C₈₋₁₈-Alkylsulfatsalze, insbesondere das Natriumsalz von Laurylsulfat (INCI: Sodium Lauryl Sulfate).

Zur Erzielung eines besonders gleichmäßigen Färbeergebnisses hat es sich ebenfalls als bevorzugt herausgestellt, wenn das in dem Verfahren eingesetzte Vorbehandlungsmittel (v) eine Mischung aus mindestens zwei anionischen Tensiden umfasst. Im Rahmen dieser Ausführungsform ist es bevorzugt, wenn das erste anionische Tensid ausgewählt ist aus der Gruppe der C₈₋₁₈-Alkylsulfate und das zweite anionische Tensid ausgewählt ist aus der Gruppe der C₈₋₁₈-Alkylpolyglykolethersulfate mit 1 bis 12 Ethoxyeinheiten.

Entsprechend ist ein Verfahren bevorzugt bei dem das Vorbehandlungsmittel (v) enthält:
(v1) mindestens zwei anionische Tenside, umfassend ein C₈₋₁₈-Alkylsulfatsalz und ein C₈₋₁₈-Alkylpolyglykolethersulfatsalz mit 1 bis 12 Ethoxyeinheiten.

In einer ganz besonders bevorzugten Ausführungsform des Verfahrens enthält das Vorbehandlungsmittel (v) eine Verbindung mit der INCI-Bezeichnung Sodium Lauryl Sulfate und/oder eine Verbindung mit der INCI-Bezeichnung Sodium Laureth Sulfate.

Das Vorbehandlungsmittel (v) enthält das mindestens eine anionische Tensid in einer bevorzugten Menge von 0,5 bis 30 Gew.-%, mehr bevorzugt von 1 bis 25 und insbesondere bevorzugt von 2 bis 20 Gew.-%, wobei sich die Mengenangaben auf das Gesamtgewicht des Vorbehandlungsmittels (v) beziehen.

### Weitere Tenside im Vorbehandlungsmittel (v)

Das Vorbehandlungsmittel kann weitere Tenside, insbesondere amphotere Tenside, umfassen.

Amphotere Tenside, welche auch als zwitterionische Tenside bezeichnet werden, werden solche oberflächenaktiven Verbindungen genannt, die im Molekül mindestens eine quartäre Ammoniumgruppe und mindestens eine -COO⁻ - oder -SO₃⁻ -Gruppe tragen. Unter amphoteren/zwitterionischen Tensiden werden auch solche oberflächenaktiven Verbindungen verstanden, die außer einer C₈- C₂₄-Alkyl- oder -Acylgruppe mindestens eine freie Aminogruppe und mindestens eine -COOH- oder -SO₃H-Gruppe enthalten und zur Ausbildung innerer Salze befähigt sind.

Gemäß einer bevorzugten Ausführungsform des Verfahrens enthält das Vorbehandlungsmittel (v) ferner mindestens ein amphoteres Tensid. Bevorzugt sind die amphoteren Tenside in dem Vorbehandlungsmittel ausgewählt aus der Gruppe bestehend aus
- Alkylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylamphodiacetat oder Alkylamphodiacetat, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe, mit einem Alkali- oder Erdalkalimetallgegenion, und
- Alkylamidopropylbetain, umfassend mindestens eine gesättigte oder ungesättigte, verzweigte oder unverzweigte C₆ bis C₂₂, bevorzugt C₁₀ bis C₁₈, bevorzugter C₁₂ bis C₁₆ Alkylgruppe.

Zu den insbesondere geeigneten amphoteren/zwitterionischen Tensiden zählen die unter der INCI-Bezeichnung bekannten amphoterenTenside Cocamidopropylbetain und Disodium Cocoamphodiacetate.

Das Vorbehandlungsmittel kann ferner nichtionische Tenside umfassen. Gemäß einer bevorzugten Ausführungsform des Verfahrens enthält das Vorbehandlungsmittel (v) ferner mindestens ein nichtionisches Tensid ausgewählt aus der Gruppe bestehend aus
- Alkylglucamid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylfructosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylglucosid, umfassend eine gesättigte oder ungesättigte, verzweigte oder unverzweigte C₆ bis C₂₂, bevorzugt C₁₀ bis C₁₈, bevorzugter C₁₂ bis C₁₆ Alkylgruppe,
- Alkylalkoholalkoxylat der Formel R₁₀(OR₁₁)ₘOH, in der R₁₀ eine lineare oder verzweigte C₆ bis C₂₂, bevorzugt C₁₀ bis C₁₈, bevorzugter C₁₂ bis C₁₆ Alkylgruppe, R₁₁ eine C₂ bis C₄, bevorzugt eine C₂ Alkylengruppe, und m 1 bis 10, bevorzugt 2 bis 8, darstellen, und
- Alkylester der Formel R₁₂COOR₁₃, in der R₁₂ eine lineare oder verzweigte C₆ bis C₂₂, bevorzugt C₁₀ bis C₁₈, bevorzugter C₁₂ bis C₁₆ Alkylgruppe, R₁₃ eine C₁ bis C₄, bevorzugt eine C₂ Alkylgruppe, darstellen.

Weitere geeignete nichtionische Tenside umfassen PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil, PEG-40 Hydrogenated Castor Oil oder PEG-60 Hydrogenated Castor Oil.

Das Vorbehandlungsmittel (v) enthält die weiteren Tenside in einer bevorzugten Menge von 0,1 bis 15 Gew.-%, mehr bevorzugt von 0,5 bis 12 und insbesondere bevorzugt von 1 bis 10 Gew.-%, wobei sich die Mengenangaben auf das Gesamtgewicht des Vorbehandlungsmittels (v) beziehen.

Das Vorbehandlungsmittel (v) enthält vorzugsweise einen wässrigen Träger für das mindestens eine anionische Tensid.

### pH-Wert des Vorbehandlungsmittels (v)

Es hat sich als bevorzugt herausgestellt, wenn das Vorbehandlungsmittel (v) einen alkalischen pH-Wert besitzt. Entsprechend weist das Vorbehandlungsmittel (v) einen pH-Wert im Bereich von 9,8 bis 10,2 auf.

Bevorzugt ist der pH-Wert des Vorbehandlungsmittels (v) 10.

Zur Einstellung des pH-Wertes kann das Vorbehandlungsmittel (v) ferner mindestens ein Alkalisierungsmittel enthalten.

Besonders bevorzugt wird das Alkalisierungsmittel ausgewählt aus der Gruppe aus Ammoniak, C₂-C₆-Alkanolaminen, basischen Aminosäuren, Alkalimetallhydroxiden und Erdalkalimetallhydroxiden.

Im Rahmen einer weiteren besonders bevorzugten Ausführungsform ist ein Verfahren dadurch gekennzeichnet, dass das Vorbehandlungsmittel (v) mindestens ein Alkalisierungsmittel enthält, das ausgewählt ist aus der Gruppe aus Ammoniak, C₂-C₆-Alkanolaminen, basischen Aminosäuren, Alkalimetallhydroxiden, Erdalkalimetallhydroxiden, Alkalimetallsilikaten, Alkalimetallmetasilikaten, Erdalkalimetallsilikaten, Erdalkalimetallmetasilikaten, Alkalimetallcarbonaten und Erdalkalimetallcarbonaten.

Es hat sich herausgestellt, dass die Behandlung mit einem Vorbehandlungsmittels(v), das Ammoniak enthält, einen besonders guten Einfluss auf die Verbesserung der Waschechtheit und der Reibechtheit der im Verfahren erhaltenen Färbungen ausübt.

Im Rahmen einer weiteren ganz besonders bevorzugten Ausführungsform ist ein Verfahren dadurch gekennzeichnet, dass das Vorbehandlungsmittel (v) Ammoniak enthält.

Ebenfalls gute Ergebnisse konnten erhalten werden, wenn das Vorbehandlungsmittel (v) mindestens ein C₂-C₆-Alkanolamin enthielt.

Die in dem Vorbehandlungsmittel (v) einsetzbaren Alkanolamine können beispielsweise ausgewählt werden aus der Gruppe der primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

In einer weiteren bevorzugten Ausführungsform ist ein erfindungsgemäßes Verfahren dadurch gekennzeichnet, dass das Vorbehandlungsmittel (v) mindestens ein Alkalisierungsmittel aus der Gruppe der Alkanolamine enthält, das bevorzugt ausgewählt ist aus der Gruppe aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol und 2-Amino-2-methylpropan-1,3-diol.

Ebenfalls gute Ergebnisse konnten erhalten werden, wenn das Vorbehandlungsmittel (v) als Versiegelungsreagenz (B1) mindestens eine basische Aminosäure enthielt.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist das Verfahren daher dadurch gekennzeichnet, dass es sich bei dem um Alkalisierungsmittel, umfassend eine basische Aminosäure aus der Gruppe Arginin, Lysin, Ornithin und/oder Histidin handelt.

In einer weiteren bevorzugten Ausführungsform ist das Verfahren dadurch gekennzeichnet, dass das Vorbehandlungsmittel (v) mindestens ein Alkalisierungsmittel aus der Gruppe der basischen Aminosäuren enthält, das bevorzugt ausgewählt ist aus der Gruppe aus Arginin, Lysin, Ornithin und Histidin.

Ebenfalls gute Ergebnisse konnten erhalten werden, wenn das Vorbehandlungsmittel (v) mindestens ein Alkalimetallhydroxid enthält. Als gut geeignete Alkalimetallhydroxide können beispielsweise Natriumhydroxid und Kaliumhydroxid genannt werden.

Ebenfalls gute Ergebnisse konnten erhalten werden, wenn das Vorbehandlungsmittel (v) ein Alkalisierungsmittel, umfassend mindestens ein Erdalkalimetallhydroxid enthielt. Als gut geeignete Erdalkalimetallhydroxide können beispielsweise Magnesiumhydroxid, Calciumhydroxid und Bariumhydroxid genannt werden.

Ebenfalls gute Ergebnisse konnten erhalten werden, wenn das Vorbehandlungsmittel (v) mindestens ein Alkalimetallsilikat und/oder Alkalimetallmetasilikat enthielt. Geeignete Alkalimetallsilikate sind beispielsweise Natriumsilikat und Kaliumsilikat. Geeignete Alkalimetallmetasilikate sind beispielsweise Natriummetasilikat und Kaliummetasilikat.

Ebenfalls gute Ergebnisse konnten erhalten werden, wenn das Vorbehandlungsmittel (v) mindestens ein Alkalimetallcarbonat und/oder Erdalkalimetallcarbonat enthielt. Geeignete Alkalimetallcarbonate sind beispielsweise Natriumcarbonat und Kaliumcarbonat. Geeignete Erdalkalimetallcarbonate sind beispielsweise Magnesiumcarbonat und Calciumcarbonat.

Innerhalb der Gruppe der vorgenannten Alkalisierungsmittel haben sich Ammoniak, C₂-C₆-Alkanolaminene, basischen Aminosäuren und Alkalimetallhydroxide als ganz besonders gut geeignet herausgestellt.

Im Rahmen einer weiteren besonders bevorzugten Ausführungsform ist das Verfahren dadurch gekennzeichnet, dass das Vorbehandlungsmittel (v) mindestens ein Alkalisierungsmittel ausgewählt aus der Gruppe aus Ammoniak, C₂-C₆-Alkanolaminenen, basischen Aminosäuren und Alkalimetallhydroxiden umfasst.

Im Rahmen einer weiteren besonders bevorzugten Ausführungsform ist das Verfahren dadurch gekennzeichnet, dass das Vorbehandlungsmittel (v) mindestens ein Alkalisierungsmittel enthält, das ausgewählt ist aus der Gruppe aus Ammoniak, 2-Aminoethan-1-ol, 3-Aminopropan-1-ol, 4-Amino-butan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid und Kaliumhydroxid.

### Mittel (a) und (b)

Im Rahmen des beschriebenen Verfahrens werden die Mittel (a) und (b) im Anschluss an das Vorbehandlungsmittel (v) auf dem keratinischen Material, insbesondere den menschlichen Haaren, appliziert. Die zwei Mittel (a) und (b) sind voneinander verschieden.

### Mittel (a)

Bevorzugt enthält das Mittel (a) den erfindungswesentlichen Inhaltsstoff (a1) in einem kosmetischen Träger, besonders bevorzugt in einem wässrigen oder wässrig-alkoholischen kosmetischen Träger. Dieser kosmetische Träger kann flüssig, gelartig oder cremeförmig sein. Auch pastöse, feste oder pulverförmige kosmetische Träger können für die Herstellung des Mittels (a) verwendet werden. Zum Zwecke der Haarbehandlung, insbesondere Haarfärbung, sind solche Träger beispielsweise Cremes, Emulsionen, Gele oder auch Tensid-haltige schäumende Lösungen, wie beispielsweise Shampoos, Schaumaerosole, Schaum-formulierungen oder andere Zubereitungen, die für die Anwendung auf dem Haar geeignet sind.

Bevorzugt enthält der kosmetische Träger - bezogen auf sein Gewicht - mindestens 2 Gew.-% Wasser. Weiter bevorzugt liegt der Wassergehalt oberhalb von 10 Gew.-%, noch weiter bevorzugt oberhalb von 20 Gew.-% und besonders bevorzugt oberhalb von 40 Gew.-%. Der kosmetische Träger kann auch wässrig-alkoholisch sein. Unter wässrig-alkoholischen Lösungen sind im Sinne der vorliegenden Erfindung wässrige Lösungen enthaltend 2 bis 70 Gew.-% eines C₁-C₄-Alkohols, insbesondere Ethanol bzw. Isopropanol, zu verstehen. Die Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

### Organische Siliciumverbindungen aus der Gruppe der Silane (a1)

Als erfindungswesentlichen Inhaltsstoff (a1) enthält das Mittel (a) mindestens eine organische Siliciumverbindung aus der Gruppe der Silane mit einem, zwei oder drei Siliciumatomen.

Besonders bevorzugt enthält das Mittel (a) mindestens eine organische Siliciumverbindung (a1), die aus Silanen mit einem, zwei oder drei Siliciumatomen ausgewählt ist, wobei die organische Siliciumverbindung eine oder mehrere Hydroxylgruppen und/oder hydrolysierbare Gruppen pro Molekül umfasst.

Diese im Mittel (a) enthaltenen organischen Siliciumverbindungen (a1) bzw. organischen Silane sind reaktive Verbindungen.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das SiliciumAtom geknüpft ist. Die erfindungsgemäßen organische Siliciumverbindungen sind Verbindungen, die eine bis drei Siliciumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Im Rahmen einer besonders bevorzugten Ausführungsform ist ein Verfahren gekennzeichnet durch die Anwendung eines Mittels (a) auf dem keratinischem Material, wobei das Mittel (a) mindestens eine organische Siliciumverbindung (a1) enthält, die aus Silanen mit einem, zwei oder drei Siliciumatomen ausgewählt ist, wobei die organische Siliciumverbindung außerdem eine oder mehrere Hydroxylgruppen oder hydrolysierbare Gruppen pro Molekül umfasst.

Im Rahmen einer ganz besonders bevorzugten Ausführungsform ist ein Verfahren gekennzeichnet durch die Anwendung eines Mittels (a) auf dem keratinischem Material, wobei das Mittel (a) mindestens eine organische Siliciumverbindung (a1) enthält, die aus Silanen mit einem, zwei oder drei Siliciumatomen ausgewählt ist, wobei die organische Siliciumverbindung außerdem eine oder mehrere basische chemische Funktionen und eine oder mehrere Hydroxylgruppen oder hydrolysierbare Gruppen pro Molekül umfasst.

Bei dieser basischen Gruppe oder basischen chemischen Funktion kann es sich beispielsweise um eine Aminogruppe, eine Alkylaminogruppe, eine Dialkylaminogruppe oder um eine Trialkylaminogruppe handeln, die bevorzugt über einen Linker mit einem Siliciumatom verbunden ist. Bevorzugt handelt es sich bei der basischen Gruppe um eine Aminogruppe, eine C₁-C₆-Alkylaminogruppe oder um eine Di(C₁-C₆)alkylaminogruppe.

Bei der oder den hydrolysierbaren Gruppen handelt es sich bevorzugt um eine C₁-C₆-Alkoxygruppe, insbesondere um eine Ethoxygruppe oder um eine Methoxygruppe. Es ist bevorzugt, wenn die hydrolysierbare Gruppe direkt an das Siliciumatom gebunden vorliegt. Handelt es sich beispielsweise bei der hydrolysierbaren Gruppe um eine Ethoxygruppe, so enthält die organische Siliciumverbindung bevorzugt eine Struktureinheit R'R"R‴Si-O-CH₂-CH₃. Die Reste R', R" und R‴ stellen hierbei die drei übrigen freien Valenzen des Siliciumatoms dar.

Ein ganz besonders bevorzugtes Verfahren ist dadurch gekennzeichnet, dass das Mittel (a) mindestens eine organische Siliciumverbindung enthält, die aus Silanen mit einem, zwei oder drei Siliciumatomen ausgewählt ist, wobei die organische Siliciumverbindung bevorzugt eine oder mehrere basische chemische Funktionen und eine oder mehrere Hydroxylgruppen oder hydrolysierbare Gruppen pro Molekül umfasst.

Ganz besonders gute Ergebnisse konnten erhalten werden, wenn das Mittel (a) mindestens eine organische Siliciumverbindung (a1) der Formel (I) und/oder (II) enthält.

Die Verbindungen der Formeln (I) und (II) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

In einer weiteren ganz besonders bevorzugten Ausführungsform ist das Verfahren dadurch gekennzeichnet, dass auf dem keratinischen Material (oder den menschlichen Haaren) ein Mittel angewendet wird, wobei das Mittel (a) mindestens eine organische Siliciumverbindung (a) der Formel (I) und/oder (II) enthält,

R₁R₂N-L-Si(OR₃)ₐ(R₄)_{b} (I),

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder eine C₁-C₆-Alkylgruppe steht,
- R₄ für eine C₁-C₆-Alkylgruppe steht
- a, für eine ganze Zahl von 1 bis 3 steht, und
- b für die ganze Zahl 3 - a steht,

   (R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
wobei
- R₅, R_{5'}, R_{5"} unabhängig voneinander für ein Wasserstoffatom oder für eine C₁-C₆-Alkylgruppe stehen,
- R₆, R_{6'} und R_{6"} unabhängig voneinander für eine C₁-C₆-Alkylgruppe stehen,
- A, A', A", A‴ und Aʺʺ unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen

   - (Aʺʺ)-Si(R₆")_{d}"(OR₅")_{c}" (III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist.

Die Substituenten R₁, R₂, R₃, R₄, R₅, R₅', R₅", R₆, R₆', R₆", R₇, R₈, L, A, A', A", A‴ und Aʺʺ in den Verbindungen der Formel (I) und (II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L-der für eine lineare oder verzweigte, zweiwertige C₁-C₂₀-Alkylengruppe steht.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung L zwei Bindungen eingehen kann. Eine Bindung erfolgt von der Aminogruppe R₁R₂N zum Linker L, und die zweite Bindung besteht zwischen dem Linker L und dem Siliciumatom.

Bevorzugt steht -L- für eine lineare, zweiwertige (d.h. divalente) C₁-C₂₀-Alkylengruppe. Weiter bevorzugt steht -L- für eine lineare zweiwertige C₁-C₆-Alkylengruppe. Besonders bevorzugt steht -L-für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die lineare Propylengruppe (-CH₂-CH₂-CH₂-) kann alternativ auch als Propan-1,3-diylgruppe bezeichnet werden.

Die organischen Siliciumverbindungen der Formel (I)

R₁R₂N-L-Si(OR₃)ₐ(R₄)_{b} (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Besonders widerstandsfähige Filme konnten erzeugt werden, wenn das Mittel (a) mindestens eine organische Siliciumverbindung (a1) der Formel (I) enthält, in welcher die Reste R₃, R₄ unabhängig voneinander für eine Methylgruppe oder für eine Ethylgruppe stehen.

Bei Einsatz des Verfahrens zum Färben von keratinischen Material konnten analog dann Färbungen mit den besten Waschechtheiten erhalten werden, wenn das Mittel (a) mindestens eine organische Siliciumverbindung der Formel (I) enthält, in welcher die Reste R₃, R₄ unabhängig voneinander für eine Methylgruppe oder für eine Ethylgruppe stehen.

Weiterhin konnten Färbungen mit den besten Waschechtheiten erhalten werden, wenn das Mittel (a) mindestens eine organische Siliciumverbindung der Formel (I) enthält, in welcher der Rest a für die Zahl 3 steht. In diesem Fall steht der Rest b für die Zahl 0.

In einer weiteren bevorzugten Ausführungsform ist das in dem Verfahren eingesetzte Mittel (a) dadurch gekennzeichnet, dass es mindestens eine organische Siliciumverbindung (a1) der Formel (I) enthält, wobei
- R₃, R₄ unabhängig voneinander für eine Methylgruppe oder für eine Ethylgruppe stehen und
- a für die Zahl 3 steht und
- b für die Zahl 0 steht.

In einer weiteren bevorzugten Ausführungsform ist ein Verfahren dadurch gekennzeichnet, dass das Mittel (a) mindestens eine organische Siliciumverbindung (a1) der Formel (I) enthält,

R₁R₂N-L-Si(OR₃)ₐ(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- R₄ für eine Methylgruppe oder für eine Ethylgruppe steht,
- a für die Zahl 3 steht und
- b für die Zahl 0 steht.

Zur Lösung der erfindungsgemäßen Aufgabenstellung besonders gut geeignete organische Siliciumverbindungen der Formel (I) sind
- (3-Aminopropyl)triethoxysilan 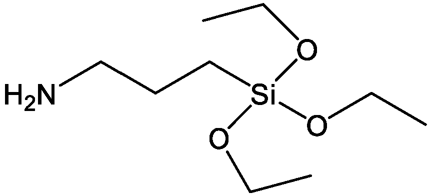
- (3-Aminopropyl)trimethoxysilan 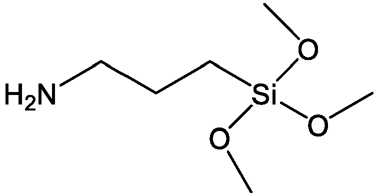
- 1-(3-Aminopropyl)silantriol 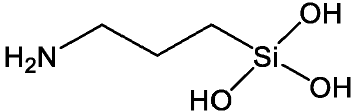
- (2-Aminoethyl)triethoxysilan 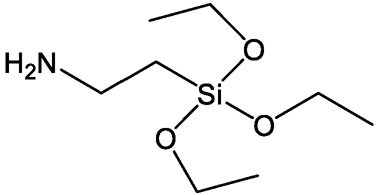
- (2-Aminoethyl)trimethoxysilan 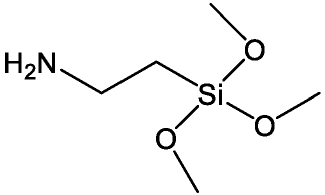 1-(2-Aminoethyl)silantriol 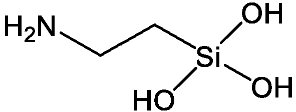
- (3-Dimethylaminopropyl)triethoxysilan 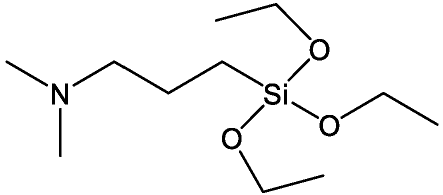
- (3-Dimethylaminopropyl)trimethoxysilan 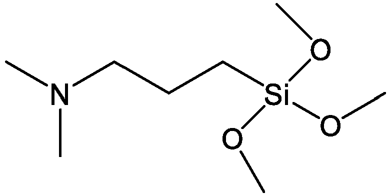 1-(3-Dimethylaminopropyl)silantriol 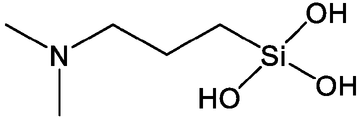
- (2-Dimethylaminoethyl)triethoxysilan. 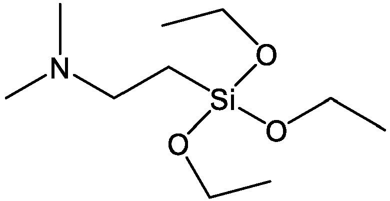
- (2-Dimethylaminoethyl)trimethoxysilan und 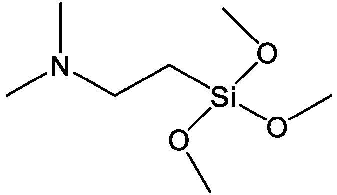 1-(2-Dimethylaminoethyl)silantriol

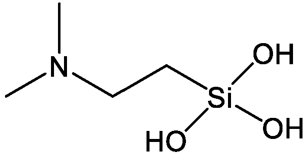

In einer weiteren bevorzugten Ausführungsform ist ein Verfahren dadurch gekennzeichnet, dass das Mittel (a) mindestens eine organische Siliciumverbindung (a1) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan und/oder
- 1-(2-Dimethylaminoethyl)silantriol.

Die vorgenannten organischen Siliciumverbindungen der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Im Rahmen einer weiteren Ausführungsform enthält das Mittel mindestens eine organische Siliciumverbindung (a1) der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II).

Die siliciumorganischen Verbindungen der Formel (II) tragen jeweils an ihren beiden Enden die Silicium-haltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Filme mit der höchsten Stabilität bzw. Färbungen mit den besten Waschechtheiten konnten erhalten werden, wenn die Reste c und c' beide für die Zahl 3 stehen. In diesem Fall stehen d und d' beide für die Zahl 0.

In einer weiteren bevorzugten Ausführungsform ist ein Verfahren dadurch gekennzeichnet, dass das Mittel (a) mindestens eine organische Siliciumverbindung (a1) der Formel (II) enthält,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die erfindungsgemäßen organischen Siliciumverbindung der Formel (IIa)

(R₅O)₃Si-(A)ₑ-[NR₇-(A')]_{f}-[O-(A")]_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ-sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der Waschechtheit erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die erfindungsgemäßen organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und Aʺʺ stehen unabhängig voneinander für eine lineare oder verzweigte, zweiwertige C₁-C₂₀-Alkylengruppe. Bevorzugt stehen die Reste A, A', A", A‴ und Aʺʺ unabhängig voneinander für eine lineare, zweiwertige C₁-C₂₀-Alkylengruppe. Weiter bevorzugt stehen die Reste A, A', A", A‴ und Aʺʺ unabhängig voneinander für eine lineare zweiwertige C₁-C₆-Alkylengruppe. Besonders bevorzugt stehen die Reste A, A', A", A‴ und Aʺʺ unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und Aʺʺ für eine Propylengruppe (-CH₂-CH₂-CH₂-).

Die zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung A, A', A", A‴ und Aʺʺ zwei Bindungen eingehen kann.

Die lineare Propylengruppe (-CH₂-CH₂-CH₂-) kann alternativ auch als Propan-1,3-diylgruppe bezeichnet werden.

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (III)

- (Aʺʺ)-Si(R₆")_{d}"(OR₅")_{c}" (III).

Ganz besonders bevorzugt stehen die Reste R7 und R8 unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)]. Steht nun der Rest R7 für eine Gruppierung der Formel (III), so enthält das Mittel (a) eine organische Siliciumverbindung mit 3 reaktiven Silan-Gruppen.

In einer weiteren bevorzugten Ausführungsform ist ein Verfahren dadurch gekennzeichnet, dass das Mittel (a) mindestens eine organische Siliciumverbindung (a1) der Formel (II) enthält,

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c} (II),

wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform ist ein Verfahren dadurch gekennzeichnet, dass das Mittel (a) mindestens eine organische Siliciumverbindung der Formel (II) enthält, wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der erfindungsgemäßen Aufgabenstellung gut geeignete organische Siliciumverbindungen der Formel (II) sind
- 3-(Trimethoxysilyl)-N-[3-(trimethoxysilyl)propyl]-1-propanamin 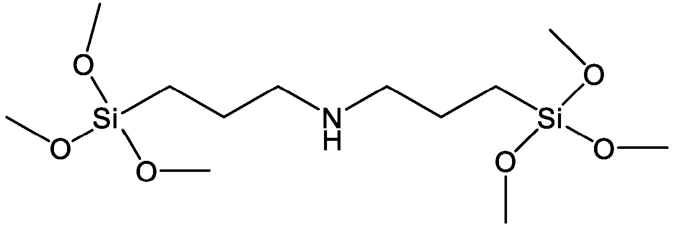
- 3-(Triethoxysilyl)-N-[3-(triethoxysilyl)propyl]-1-propanamin 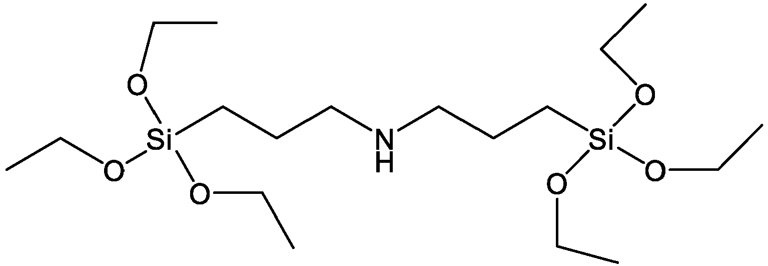
- N-Methyl-3-(trimethoxysilyl)-N-[3-(trimethoxysilyl)propyl]-1-propanamin 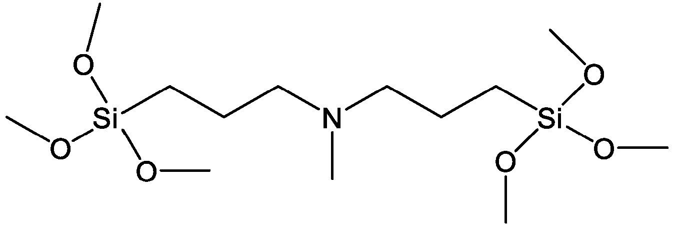
- N-Methyl-3-(triethoxysilyl)-N-[3-(triethoxysilyl)propyl]-1-propanamin 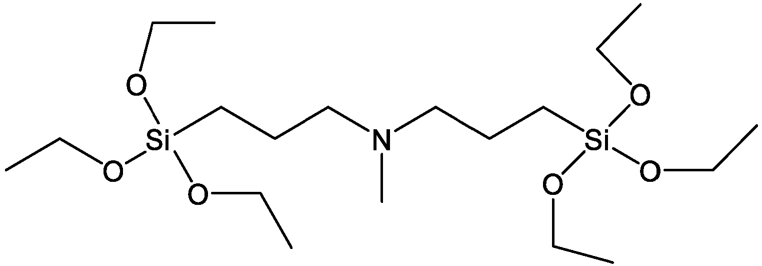
- 2-[Bis[3-(trimethoxysilyl)propyl]amino]-ethanol 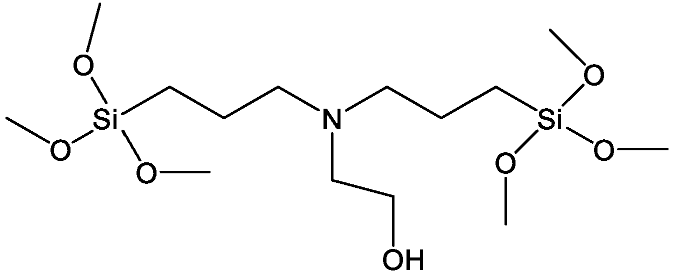
- 2-[Bis[3-(triethoxysilyl)propyl]amino]-ethanol 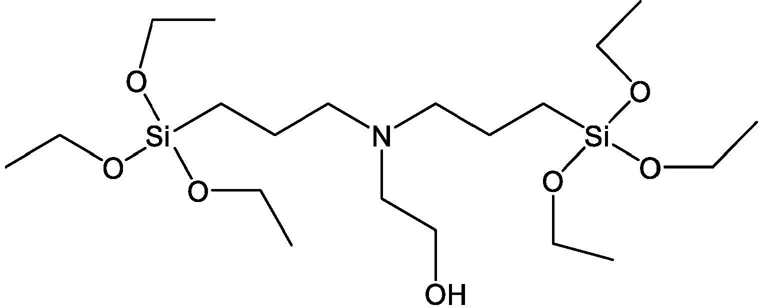
- 3-(Trimethoxysilyl)-N,N-bis[3-(trimethoxysilyl)propyl]-1-propanamin 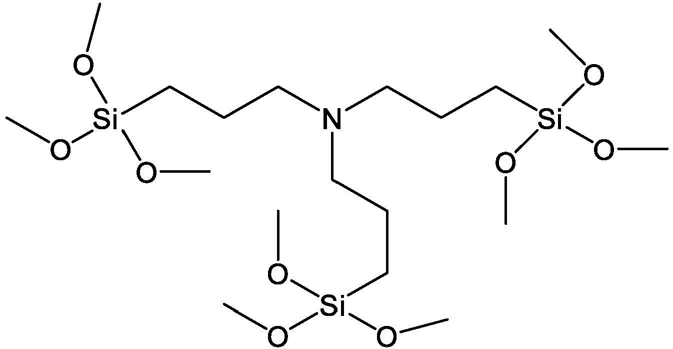
- 3-(Triethoxysilyl)-N,N-bis[3-(triethoxysilyl)propyl]-1-propanamin 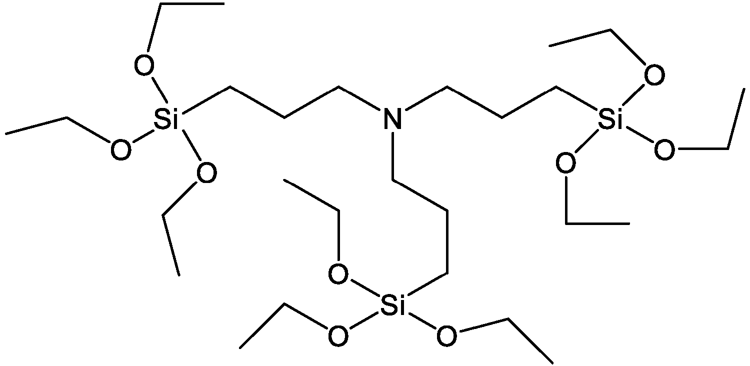
- N1,N1-Bis[3-(trimethoxysilyl)propyl]-1,2-ethanediamin, 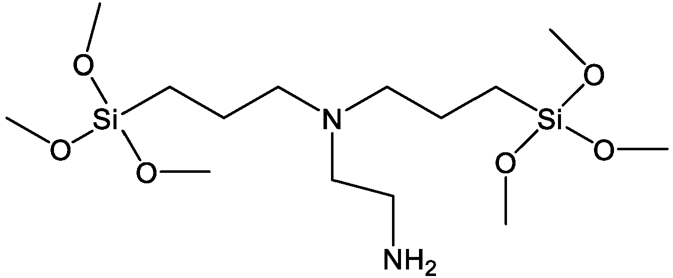
- N1,N1-Bis[3-(triethoxysilyl)propyl]-1,2-ethanediamin, 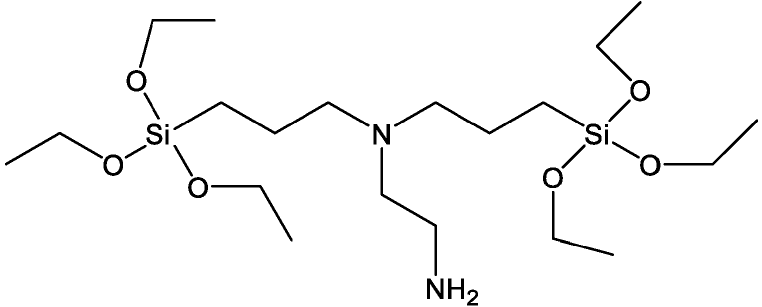
- N,N-Bis[3-(trimethoxysilyl)propyl]-2-propen-1-amin 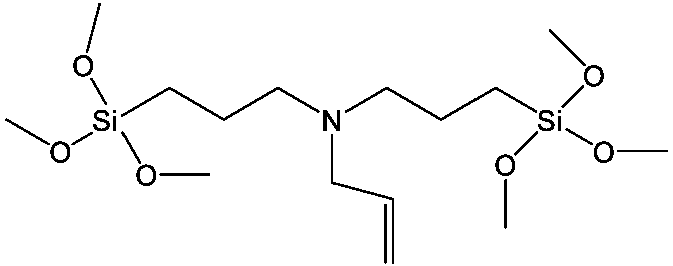
- N,N-Bis[3-(triethoxysilyl)propyl]-2-propen-1-amin

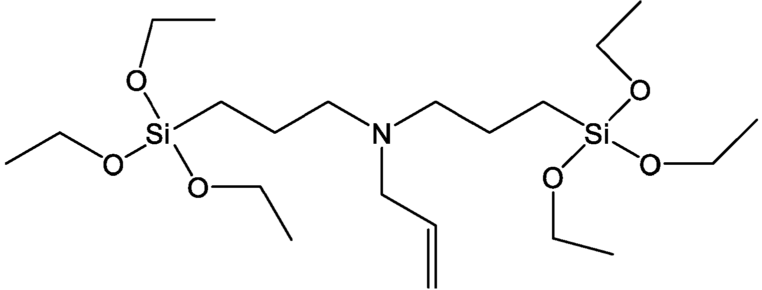

Die vorgenannten organische Siliciumverbindung der Formel (II) sind kommerziell erhältlich. Bis(trimethoxysilylpropyl)amine mit der CAS-Nummer 82985-35-1 kann beispielsweise von Sigma-Aldrich käuflich erworben werden.

Bis[3-(triethoxysilyl)propyl]amine mit der CAS-Nummer 13497-18-2 kann zum Beispiel von Sigma-Aldrich käuflich erworben werden.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In einer weiteren bevorzugten Ausführungsform ist ein Verfahren dadurch gekennzeichnet, dass das Mittel (a) mindestens eine organische Siliciumverbindung (a1) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin.

In weiteren Versuchen, insbesondere Färbeversuchen, hat es sich ebenfalls als ganz besonders vorteilhaft herausgestellt, wenn das in dem Verfahren auf dem keratinischen Material angewendete Mittel (a) mindestens eine organische Siliciumverbindung der Formel (IV) enthält

R₉Si(OR₁₀)ₖ(R₁₁)ₘ (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkyl-alkoxy-silane oder der Alkyl-hydroxy-silane bezeichnet werden,

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist das Verfahren dadurch gekennzeichnet, dass das Mittel (a) mindestens eine organische Siliciumverbindung (a1) der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein Verfahren dadurch gekennzeichnet, dass das Mittel (a) zusätzlich zu der oder den organischen Siliciumverbindungen der Formel (I) mindestens eine weitere organische Siliciumverbindung der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein Verfahren dadurch gekennzeichnet, dass das Mittel (a) zusätzlich zu der oder den organischen Siliciumverbindungen der Formel (II) mindestens eine weitere organische Siliciumverbindung der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In einer weiteren bevorzugten Ausführungsform ist ein Verfahren dadurch gekennzeichnet, dass das Mittel (a) zusätzlich zu der oder den organischen Siliciumverbindungen der Formel (I) und/oder (II) mindestens eine weitere organische Siliciumverbindung der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₈-Alkylgruppe. Diese C₁-C₁₈-Alkylgruppe ist gesättigt und kann linear oder verzweigt sein. Bevorzugt steht R₉ für eine lineare C₁-C₁₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe, eine n-Dodecylgruppe oder eine n-Octyldecylgruppe. Besonders bevorzugt steht R₉ für eine Methylgruppe, eine Ethylgruppe, eine n-Hexylgruppe oder eine n-Octylgruppe.

In den organischen Siliciumverbindungen der Form (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen der Form (IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Besonders stabile Filme, d.h. Färbungen mit besonders guten Waschechtheiten konnten erhalten werden, wenn im Verfahren ein Mittel (a) eingesetzt wurde, welches mindestens eine organische Siliciumverbindung (a1) der Formel (IV) enthält, in welcher der Rest k für die Zahl 3 steht. In diesem Fall steht der Rest m für die Zahl 0.

Zur Lösung der erfindungsgemäßen Aufgabenstellung besonders gut geeignete organische Siliciumverbindungen der Formel (IV) sind
- Methyltrimethoxysilan 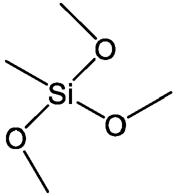
- Methyltriethoxysilan 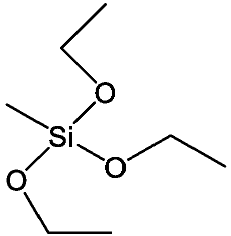
- Ethyltrimethoxysilan 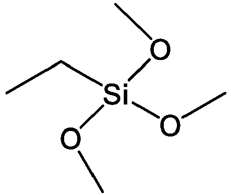
- Ethyltriethoxysilan 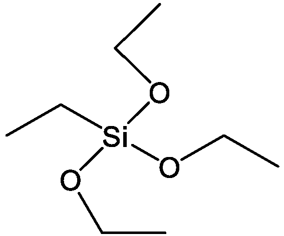
- n-Hexyltrimethoxysilan 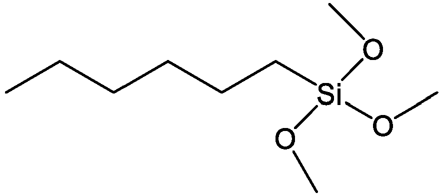
- n-Hexyltriethoxysilan 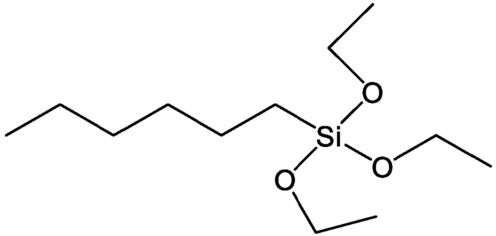
- n-Octyltrimethoxysilan 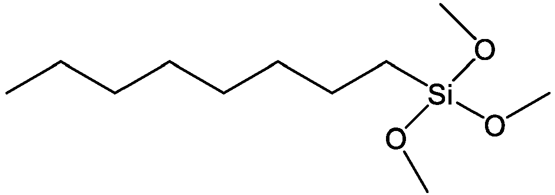
- n-Octyltriethoxysilan 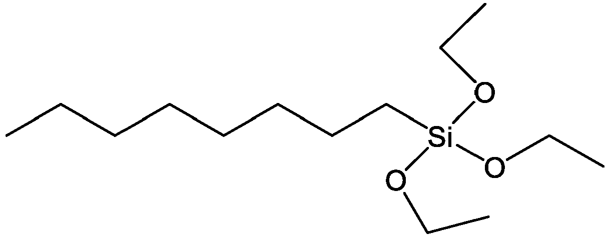
- n-Dodecyltrimethoxysilan und/oder 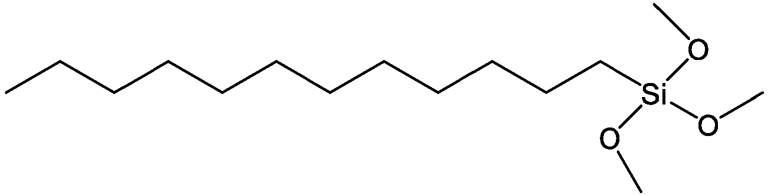
- n-Dodecyltriethoxysilan.

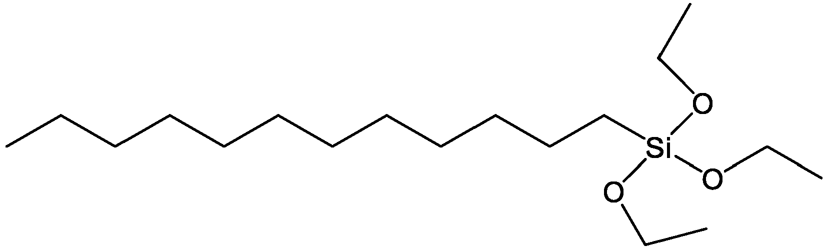

In einer weiteren bevorzugten Ausführungsform ist ein Verfahren dadurch gekennzeichnet, dass das Mittel (a) mindestens eine organische Siliciumverbindung (a1) der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan
- Dodecyltriethoxysilan
- Octadecyltrimethoxysilan und/oder
- Octadecyltriethoxysilan.

Bei den zuvor beschriebenen organischen Siliciumverbindungen handelt es sich um reaktive Verbindungen. In diesem Zusammenhang hat es sich als bevorzugt herausgestellt, wenn das Mittel (a) - bezogen auf das Gesamtgewicht des Mittels (a) - eine oder mehrere organische Siliciumverbindungen (a1) in einer Gesamtmenge von 0,1 bis 20 Gew.-%, bevorzugt 1 bis 15 Gew.-% und besonders bevorzugt 2 bis 8 Gew.-% enthält.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen (a1) in einer Gesamtmenge von 0,1 bis 20 Gew.-%, bevorzugt 1 bis 15 Gew.-% und besonders bevorzugt 2 bis 8 Gew.-% enthält.

Zur Erzielung von besonders guten Färbeergebnissen ist es insbesondere von Vorteil, die organische Siliciumverbindungen der Formel (I) und/oder (II) in bestimmten Mengenbereichen im Mittel (a) einzusetzen. Besonders bevorzugt enthält das Mittel (a) - bezogen auf das Gesamtgewicht des Mittels (a) - ein oder mehrere organische Siliciumverbindungen der Formel (I) und/oder (II) in einer Gesamtmenge von 0,1 bis 10 Gew.-%, bevorzugt 0,5 bis 5 Gew.-% und besonders bevorzugt 0,5 bis 3 Gew.-%.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen der Formel (I) und/oder (II) in einer Gesamtmenge von 0,1 bis 10 Gew.-%, bevorzugt 0,5 bis 5 Gew.-% und besonders bevorzugt 0,5 bis 3 Gew.-% enthält.

Weiterhin hat es sich als ganz besonders bevorzugt herausgestellt, wenn auch das oder die organische Siliciumverbindungen der Formel (IV) in bestimmten Mengenbereichen im Mittel (a) enthalten sind. Besonders bevorzugt enthält das Mittel (a) - bezogen auf das Gesamtgewicht des Mittels (a) - eine oder mehrere organische Siliciumverbindungen der Formel (IV) in einer Gesamtmenge von 0,1 bis 20 Gew.-%, bevorzugt 2 bis 15 Gew.-% und besonders bevorzugt 4 bis 9 Gew.-%.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen der Formel (IV) in einer Gesamtmenge von 0,1 bis 20 Gew.-%, bevorzugt 2 bis 15 Gew.-% und besonders bevorzugt 3,2 bis 10 Gew.-% enthält.

Im Zuge der zu dieser Erfindung führenden Arbeiten hat sich herausgestellt, dass auf dem keratinischen Material auch dann besonders stabile und gleichmäßige Filme erhalten werden konnten, wenn das Mittel (a) zwei strukturell voneinander verschiedene organische Siliciumverbindungen enthält.

In einer weiteren bevorzugten Ausführungsform ist ein Verfahren dadurch gekennzeichnet, dass das Mittel (a) mindestens zwei strukturell voneinander verschiedene organische Siliciumverbindungen enthält.

In einer bevorzugten Ausführungsform ist ein Verfahren dadurch gekennzeichnet, dass auf dem keratinischen Material ein Mittel (a) angewendet wird, welches mindestens eine organische Siliciumverbindungen der Formel (I) und mindestens eine organische Siliciumverbindungen der Formel (IV) enthält.

In einer explizit ganz besonders bevorzugten Ausführungsform ist ein Verfahren dadurch gekennzeichnet, dass auf dem keratinischen Material ein Mittel (a) angewendet wird, welches mindestens eine organische Siliciumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Siliciumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein Verfahren dadurch gekennzeichnet, dass das Mittel (a) - bezogen auf das Gesamtgewicht des Mittels (a) - enthält:
- 0,5 bis 5 Gew.-% mindestens einer ersten organischen Siliciumverbindung (a1) die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10 Gew.-% mindestens einer zweiten organischen Siliciumverbindung (a1) die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Im Rahmen dieser Ausführungsform enthält das Mittel (a) eine oder mehrere organischen Siliciumverbindungen einer ersten Gruppe in einer Gesamtmenge von 0,5 bis 5 Gew.-%. Die organischen Siliciumverbindungen dieser ersten Gruppe sind ausgewählt aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und/oder (2-Dimethylaminoethyl)triethoxysilan.

Im Rahmen dieser Ausführungsform enthält das Mittel (a) eine oder mehrere organischen Siliciumverbindungen einer zweiten Gruppe in einer Gesamtmenge von 3,2 bis 10 Gew.-%. Die organischen Siliciumverbindungen dieser zweiten Gruppe sind ausgewählt aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem Mittel (a) enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem Mittel (a) enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Ganz besonders gute Ergebnisse konnten erhalten werden, wenn in dem Verfahren organischen Siliciumverbindungen der Formel (I) und/oder (II) eingesetzt wurden. Da wie bereits zuvor beschrieben bereits bei Spuren von Feuchtigkeit eine Hydrolyse/Kondensation einsetzt, sind auch die Hydrolyse- und/oder Kondensationsprodukte der organischen Siliciumverbindungen (I) und/oder (II) von dieser Ausführungsform mit umfasst.

### pH-Wert des Mittels (a)

Es hat sich als bevorzugt herausgestellt, wenn das Mittel (a) in Form eines wasserhaltigen Mittels konfektioniert wird, das auf einen alkalischen pH-Wert eingestellt wird.

Zur Einstellung des pH-Wertes kann das Mittel (a) mindestens ein Alkalisierungsmittel enthalten.

Zur Einstellung des gewünschten pH-Wertes können die Mittel (a) daher auch mindestens ein Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel kann das Mittel (a) beispielsweise Ammoniak, Alkanolamine und/oder basische Aminosäuren enthalten.

Die in dem Mittel einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind solche Aminosäuren zu verstehen, welche einen isoelektrischen Punkt pl von größer 7 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Obwohl die Mittel (a) bevorzugt auf pH-Werte im alkalischen Bereich eingestellt werden, kann es dennoch prinzipiell notwendig sein, zur Feinjustierung des gewünschten pH-Wertes in geringen Mengen auch Acidifizierungsmittel einzusetzen. Erfindungsgemäß geeignete Acidifizierungsmittel sind beispielsweise Zitronensäure, Milchsäure, Essigsäure oder auch verdünnte Mineralsäuren (wie beispielsweise Salzsäure, Schwefelsäure, Phosphorsäure).

Im Rahmen der zu dieser Erfindung führenden Arbeiten hat es sich jedoch herausgestellt, dass die Gegenwart des Alkalisierungsmittels bzw. die Einstellung des alkalischen pH-Wertes für die Ausbildung von resistenten Filmen auf dem keratinischen Material essentiell ist. Die Gegenwart zu großer Mengen an Säuren kann sich hierbei negativ auf die Festigkeit der Filme auswirken. Aus diesem Grund hat es sich als bevorzugt herausgestellt, die Einsatzmengen an Säuren im Mittel (a) möglichst gering zu halten. Aus diesem Grund ist es von Vorteil, wenn die Gesamtmenge der im Mittel (a) enthaltenen organischen und/oder anorganischen Säuren einen bestimmten Wert nicht überschreitet.

In einer weiteren bevorzugten Ausführungsform ist ein Verfahren dadurch gekennzeichnet, dass die Gesamtmenge der im Mittel (a) enthaltenen organischen Säuren aus der Gruppe aus Citronensäure, Weinsäure, Äpfelsäure und Milchsäure bei einem Gehalt unterhalb von 1 Gew.-%, bevorzugt unterhalb von 0,7 Gew.-%, weiter bevorzugt unterhalb von 0,5 Gew.-%, noch weiter bevorzugt unterhalb von 0,1 Gew.-% und ganz besonders bevorzugt unterhalb von 0,01 Gew.-% liegt.

In einer weiteren bevorzugten Ausführungsform ist ein Verfahren dadurch gekennzeichnet, dass die Gesamtmenge der im Mittel (a) enthaltenen anorganischen Säuren aus der Gruppe aus Salzsäure, Schwefelsäure und Phosphorsäure bei einem Gehalt unterhalb von 1 Gew.-%, bevorzugt unterhalb von 0,7 Gew.-%, weiter bevorzugt unterhalb von 0,5 Gew.-%, noch weiter bevorzugt unterhalb von 0,1 Gew.-% und ganz besonders bevorzugt unterhalb von 0,01 Gew.-% liegt.

Die zuvor angegebenen maximalen Gesamtmengen der im Mittel (a) enthaltenen Säuren ist hierbei immer auf das Gesamtgewicht des Mittels (a) bezogen.

### Mittel (b)

Das Verfahren zur Behandlung von keratinischen Material umfasst neben der Anwendung der Mittel (v) und (a) auch die Anwendung des Mittels (b). Das Mittel (b) ist dadurch gekennzeichnet, dass es mindestens ein Versiegelungsreagenz (b1) enthält.

Die Anwendung des Mittels (b) auf das mit Mittel (a) behandelte keratinische Material führt dazu, dass die in dem Verfahren erzielten Färbungen haltbarer gemacht werden. Insbesondere kann durch Anwendung des Mittels (b) die Waschechtheit und die Reibechtheit der im Verfahren erhaltenen Färbungen verbessert werden.

Das Versiegelungsreagenz (b1) umfasst eine Verbindung ausgewählt aus der Gruppe bestehend aus filmbildenden Polymeren, Alkalisierungsmitteln, Acidifizierungsmittel und Mischungen daraus.

Es kann bevorzugt sein, dass das Versiegelungsreagenz ein filmbildendes Polymer umfasst.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden Polymers als Versiegelungsreagenz (b1) nicht mehr als 10⁷ g/mol, bevorzugt nicht mehr als 10⁶ g/mol und besonders bevorzugt nicht mehr als 10⁵ g/mol beträgt.

Unter einem filmbildenden Polymer wird im Sinne der Erfindung ein Polymer verstanden, welches in der Lage ist, auf einem Substrat, beispielsweise auf einem keratinischen Material oder einer keratinischen Faser, einen Film auszubilden. Die Ausbildung eines Films kann beispielsweise durch Betrachtung des mit dem Polymer behandelten keratinischen Materials unter einem Mikroskop nachgewiesen werden.

Die filmbildenden Polymere (b1) im Mittel (b) können hydrophil oder hydrophob sein.

Im Rahmen einer ersten Ausführungsform kann es bevorzugt sein, im Mittel (b) mindestens ein hydrophobes, filmbildendes Polymer als Versiegelungsreagenz (b1) einzusetzen.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die Silikon-Polymere, die Polymere des Acrylamid-Typs und die Polyisoprene genannt werden.

Besonders gut geeignete filmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

In einer weiteren bevorzugten Ausführungsform ist ein Mittel (b) dadurch gekennzeichnet, dass es mindestens ein filmbildendes, hydrophobes Polymer als Versiegelungsreagenz (b1) enthält, das ausgewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Zur Lösung der erfindungsgemäßen Aufgabenstellung haben sich insbesondere die filmbildenden hydrophoben Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C₂-C₁₀-Hydroxyalkylgruppe.

Weitere filmbildende hydrophobe Polymere können ausgewählt sein aus den Homo- oder Copolymeren von Isooctyl(meth)acrylat, Isononyl(meth)acrylat, 2-Ethylhexyl(meth)acrylat, Lauryl(meth)acrylat, Isopentyl(meth)acrylat, n-Butyl(meth)acrylat, Isobutyl(meth)acrylat, Ethyl(meth)acrylat, Methyl(meth)acrylat, tert-Butyl(meth)acrylat, Stearyl(meth)acrylat, Hydroxyethyl-(meth)acrylat, 2-Hydroxypropyl(meth)acrylat, 3-Hydroxypropyl(meth)acrylat und/oder Gemischen hiervon.

Weitere filmbildende hydrophobe Polymere können ausgewählt sein aus den Homo- oder Copolymeren von (Meth)acrylamid, N-Alkyl(meth)acrylamiden, insbesondere solchen mit C2-C18 Alkylgruppen, wie beispielsweise N-Ethylacrylamid, N-tert-Butylacrylamid, le N-Octylacrylamid, N-Di(C1-C4)alkyl(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®} 22 (Acrylates/Steareth-20 Methacrylate Copolymer), Aculyn^{®} 28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®} (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyloxazols, des Vinylthiazols, des Vinylpyrimidins oder des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} oder LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copolymere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styren/Butylen, Styren/Isopren, Styren/Butadien. Entsprechende Polymere werden von der BASF unter dem Handelsnamen "Luvitol HSB" kommerziell vertrieben.

Überraschenderweise hat sich herausgestellt, dass dann ganz besonders intensive und waschechte Färbungen erhalten werden konnten, wenn das Mittel (b) mindestens ein filmbildendes Polymer als Versiegelungsreagenz (b1) enthielt, das ausgewählt wurde aus der Gruppe der der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

In einer weiteren bevorzugten Ausführungsform ist ein Verfahren dadurch gekennzeichnet, dass das Mittel (b) mindestens ein filmbildendes Polymer als Versiegelungsreagenz (b1) enthält, das ausgewählt ist aus der Gruppe der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

Im Rahmen einer weiteren Ausführungsform kann es bevorzugt sein, im Mittel (b) mindestens ein hydrophiles, filmbildendes Polymer als Versiegelungsreagenz (b1) einzusetzen.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint makroskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Als filmbildende, hydrophile Polymere können nichtionische, anionische und kationische Polymere eingesetzt werden.

Geeignete filmbildende, hydrophile Polymere können beispielsweise aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummis, der Polysaccharide und/oder der Acrylamid-(Co)Polymere ausgewählt werden.

Weiterhin ist es ganz besonders bevorzugt, als filmbildendes hydrophiles Polymer Polyvinylpyrrolidon (PVP) und/oder ein Vinylpyrrolidon-haltiges Copolymer einzusetzen.

In einer weiteren ganz besonders bevorzugten Ausführungsform ist ein Mittel (b) dadurch gekennzeichnet, dass es mindestens ein filmbildendes, hydrophiles Polymer als Versiegelungsreagenz (b1) enthält, das ausgewählt ist aus der Gruppe aus Polyvinylpyrrolidon (PVP) und den Copolymeren des Polyvinylpyrrolidons.

Es ist weiterhin bevorzugt, wenn das Mittel als filmbildendes, hydrophiles Polymer Polyvinylpyrrolidon (PVP) enthält. Überraschenderweise war auch die Waschechtheit der Färbungen, die mit PVPhaltigen Mitteln (b) erhalten werden konnten, sehr gut.

Besonders gut geeignete Polyvinylpyrrolidone sind beispielsweise unter der Bezeichnung Luviskol^{®} K von der BASF SE erhältlich, insbesondere Luviskol^{®} K 90 oder Luviskol^{®} K 85 der Firma BASF SE.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Der Einsatz von filmbildenden hydrophilen Polymeren aus der Gruppe der Copolymere des Polyvinylpyrrolidons als Versiegelungsreagenz (b1) hat ebenfalls zu besonders guten und waschechten Farbergebnissen geführt.

Als besonders gut geeignete filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionische Polymere.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

In einer weiteren ganz besonders bevorzugten Ausführungsform ist ein Mittel (b) dadurch gekennzeichnet, dass es mindestens ein filmbildendes, hydrophiles Polymer als Versiegelungsreagenz (b1) enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren.

Ein weiteres geeigetes Copolymer des Vinylpyrrolidons ist das unter der INCI Bezeichnung Maltodextrin/VP Copolymer bekannte Polymer.

Weiterhin konnte intensiv gefärbtes keratinischen Material, insbesondere Haare, mit sehr guten Waschechtheiten erhalten werden, wenn als filmbildendes, hydrophiles Polymer ein nichtionisches, filmbildendes, hydrophiles Polymer eingesetzt wurde.

Im Rahmen einer weiteren Ausführungsform kann das Mittel (b) mindestens ein nichtionisches, filmbildendes, hydrophiles Polymer als Versiegelungsreagenz (b1) enthalten.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Es sind die Mittel ganz besonders bevorzugt, die als nichtionisches, filmbildendes, hydrophiles Polymer mindestens ein Polymer enthalten, das ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C1 bis C4)-Alkylamino-(C2 bis C4)-alkylacrylamid.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®} CC 10 von der Firma ISP verkauft wird.

Ein kationisches Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-Wasser-Gemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Als geeignete anionische filmbildende, hydrophile Polymere können zum Beispiel Acrylsäure-Polymere eingesetzt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Beispiele für geeignete filmbildende, hydrophile Polymere aus der Gruppe der natürlichen Gums sind Xanthan Gum, Gellan Gum, Carob Gum.

Beispiele für geeignete filmbildende, hydrophile Polymere aus der Gruppe der Polysaccharide sind Hydroxyethylcellulose, Hydroxypropylcellulose, Ethylcellulose und Carboxymethylcellulose.

Geeignete filmbildende, hydrophile Polymere aus der Gruppe der Acrylamide sind beispielsweise Polymere, welche ausgehend von Monomeren der (Meth)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure. Bevorzugte Polymere der Poly(meth)arylamido-C1-C4-alkyl-sulfonsäuren sind vernetzt und zu mindestens 90 % neutralisiert. Diese Polymere können vernetzt oder auch unvernetzt sein. Vernetzte und ganz oder teilweise neutralisierte Polymere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres bevorzugtes Polymer dieses Typs ist das der Firma Clariant unter dem Handelsnamen Hostacerin AMPS vertriebene vernetzte Poly-2-acrylamido-2methyl-propanesulphonsäure-Polymer, das teilweise mit Ammoniak neutralisiert ist.

In einer weiteren explizit ganz besonders bevorzugten Ausführungsform ist ein Verfahren dadurch gekennzeichnet, dass das Mittel (b) mindestens ein anionisches, filmbildendes, Polymer als Versiegelungsreagenz (b1) enthält.

In diesem Zusammenhang konnten die besten Ergebnisse erhalten werden, wenn das Mittel (b) mindestens ein filmbildendes Polymer als Versiegelungsreagenz (b1) enthält, das mindestens eine Struktureinheit der Formel (P-I) und mindestens eine Struktureinheit der Formel (P-II) umfasst 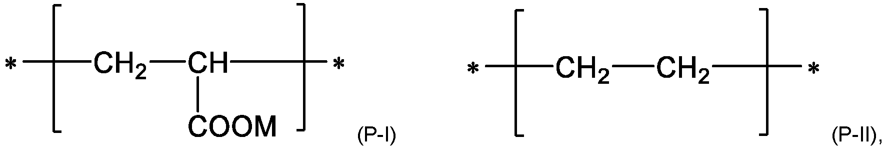 wobei
M für ein Wasserstoffatom oder für Ammonium (NH₄), Natrium, Kalium, ½ Magnesium oder ½ Calcium steht.

In einer weiteren bevorzugten Ausführungsform ist ein Verfahren dadurch gekennzeichnet, dass das Mittel (b) mindestens ein filmbildendes Polymer als Versiegelungsreagenz (b1) enthält, das mindestens eine Struktureinheit der Formel (P-I) und mindestens eine Struktureinheit der Formel (P-II) umfasst 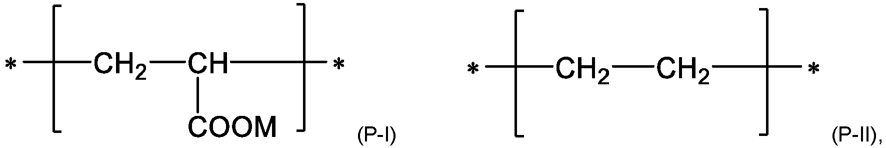 wobei
M für ein Wasserstoffatom oder für Ammonium (NH₄), Natrium, Kalium, ½ Magnesium oder ½ Calcium steht.

Wenn M für ein Wasserstoffatom steht, basiert die Struktureinheit der Formel (P-I) auf einer Acrylsäure-Einheit.

Wenn M für ein Ammonium-Gegenion steht, basiert die Struktureinheit der Formel (P-I) auf dem Ammoniumsalz der Acrylsäure.

Wenn M für ein Natrium-Gegenion steht, basiert die Struktureinheit der Formel (P-I) auf dem Natriumsalz der Acrylsäure.

Wenn M für ein Kalium-Gegenion steht, basiert die Struktureinheit der Formel (P-I) auf dem Kaliumsalz der Acrylsäure.

Wenn M für ein halbes Äquivalent eines Magnesium-Gegenions steht, basiert die Struktureinheit der Formel (P-I) auf dem Magnesiumsalz der Acrylsäure.

Wenn M für ein halbes Äquivalent eines Calcium-Gegenions steht, basiert die Struktureinheit der Formel (P-I) auf dem Calciumsalz der Acrylsäure.

Das oder die filmbildenden Polymere (b1) werden bevorzugt in bestimmten Mengenbereichen in dem Mittel (b) eingesetzt. In diesem Zusammenhang hat es sich zur Lösung der erfindungsgemäßen Aufgabenstellung als besonders bevorzugt erwiesen, wenn das Mittel (b) - bezogen auf das Gesamtgewicht des Mittels (b) - ein oder mehrere filmbildende Polymere als Versiegelungsreagenz (b1) in einer Gesamtmenge von 0,1 bis 18 Gew.-%, bevorzugt von 1 bis 16 Gew.-%, weiter bevorzugt von 5 bis 14,5 Gew.-% und ganz besonders bevorzugt von 8 bis 12 Gew.-% enthält.

In einer weiteren bevorzugten Ausführungsform ist ein Verfahren dadurch gekennzeichnet, dass das Mittel (b) - bezogen auf das Gesamtgewicht des Mittels (b) - ein oder mehrere filmbildende Polymere als Versiegelungsreagenz (b1) in einer Gesamtmenge von 0,1 bis 18 Gew.-%, bevorzugt von 1 bis 16 Gew.-%, weiter bevorzugt von 5 bis 14,5 Gew.-% und ganz besonders bevorzugt von 8 bis 12 Gew.-% enthält.

Mit der Anwendung des Mittels (b), umfassend ein filmbildendes Polymer als Versiegelungsreagenz (b1), soll der zunächst durch die Anwendung des Mittels (a) erzeugte, ggf. gefärbte Film versiegelt und/oder fixiert werden. Mit Applikation des zweiten Mittels (b) mit einem filmbildenden Polymer als Versiegelungsreagenz (b1) lagert sich auf dem im ersten Schicht erzeugten, ggf. gefärbten Film das filmbildende Polymer (b1) in Form eines weiteren Films ab. Enthält das Mittel (b) ferner mindestens eine farbgebende Verbindung wird der Farbeindruck eines im ersten Schritt hergestellten, gefärbten Films je nach verwendeter farbgebender Verbindung verstärkt oder modifiziert oder durch Ausbildung eines zweiten, gefärbten Films auf einem ersten, ungefärbten Film eine Färbung des behandelten keratinischen Materials erhalten. Das auf diese Weise erzeugte mehrschichtige Filmsystem weist eine verbesserte Widerstandsfähigkeit gegenüber äußeren Einflüssen auf.

In einer alternativen Ausführungsform enthält das Versiegelungsreagenz (b1) ein Alkalisierungsmittel.

Besonders bevorzugt wird das Alkalisierungsmittel ausgewählt aus der Gruppe aus Ammoniak, C₂-C₆-Alkanolaminen, basischen Aminosäuren, Alkalimetallhydroxiden und Erdalkalimetallhydroxiden.

Im Rahmen einer weiteren besonders bevorzugten Ausführungsform ist ein Verfahren dadurch gekennzeichnet, dass das Mittel (b) mindestens ein Alkalisierungsmittel als Versiegelungsreagenz (b1) enthält, das ausgewählt ist aus der Gruppe aus Ammoniak, C₂-C₆-Alkanolaminen, basischen Aminosäuren, Alkalimetallhydroxiden, Erdalkalimetallhydroxiden, Alkalimetallsilikaten, Alkalimetallmetasilikaten, Erdalkalimetallsilikaten, Erdalkalimetallmetasilikaten, Alkalimetallcarbonaten und Erdalkalimetallcarbonaten.

Es hat sich herausgestellt, dass die Nachbehandlung mit einem Mittel (b), das Ammoniak enthält, einen besonders guten Einfluss auf die Verbesserung der Waschechtheit und der Reibechtheit der im Verfahren erhaltenen Färbungen ausübt.

Im Rahmen einer weiteren ganz besonders bevorzugten Ausführungsform ist ein Verfahren dadurch gekennzeichnet, dass die Zusammensetzung (b) als Versiegelungsreagenz (b1) Ammoniak enthält.

Ebenfalls gute Ergebnisse konnten erhalten werden, wenn die Zusammensetzung (b) als Versiegelungsreagenz (b1) mindestens ein C₂-C₆-Alkanolamin enthielt.

Die in der Zusammensetzung (b) einsetzbaren Alkanolamine können beispielsweise ausgewählt werden aus der Gruppe der primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

In einer weiteren bevorzugten Ausführungsform ist ein erfindungsgemäßes Verfahren dadurch gekennzeichnet, dass die Zusammensetzung (b) als Versiegelungsreagenz (b1) mindestens ein Alkalisierungsmittel aus der Gruppe der Alkanolamine enthält, das bevorzugt ausgewählt ist aus der Gruppe aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol und 2-Amino-2-methylpropan-1,3-diol.

Ebenfalls gute Ergebnisse konnten erhalten werden, wenn die Zusammensetzung (b) als Versiegelungsreagenz (b1) mindestens eine basische Aminosäure enthielt.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist das Verfahren daher dadurch gekennzeichnet, dass es sich bei dem Versiegelungsreagenz (b1) um Alkalisierungsmittel, umfassend eine basische Aminosäure aus der Gruppe Arginin, Lysin, Ornithin und/oder Histidin handelt.

In einer weiteren bevorzugten Ausführungsform ist das Verfahren dadurch gekennzeichnet, dass das Mittel (b) als Versiegelungsreagenz (b1) mindestens ein Alkalisierungsmittel aus der Gruppe der basischen Aminosäuren enthält, das bevorzugt ausgewählt ist aus der Gruppe aus Arginin, Lysin, Ornithin und Histidin.

Ebenfalls gute Ergebnisse konnten erhalten werden, wenn das Mittel (b) als Versiegelungsreagenz (b1) mindestens ein Alkalimetallhydroxid enthält. Als gut geeignete Alkalimetallhydroxide können beispielsweise Natriumhydroxid und Kaliumhydroxid genannt werden.

Ebenfalls gute Ergebnisse konnten erhalten werden, wenn die Zusammensetzung (b) als Versiegelungsreagenz (b1) ein Alkalisierungsmittel, umfassend mindestens ein Erdalkalimetallhydroxid enthielt. Als gut geeignete Erdalkalimetallhydroxide können beispielsweise Magnesiumydroxid, Caliumhydroxid und Bariumhydroxid genannt werden.

Ebenfalls gute Ergebnisse konnten erhalten werden, wenn das Mittel (b) als Versiegelungsreagenz (b1) mindestens ein Alkalimetallsilikat und/oder Alkalimetallmetasilikat enthielt. Geeignete Alkalimetallsilikate sind beispielsweise Natriumsilikat und Kaliumsilikat. Geeignete Alkalimetallmetasilikate sind beispielsweise Natriummetasilikat und Kaliummetasilikat.

Ebenfalls gute Ergebnisse konnten erhalten werden, wenn das Mittel (b) als Versiegelungsreagenz (b1) mindestens ein Alkalimetallcarbonat und/oder Erdalkalimetallcarbonat enthielt. Geeignete Alkalimetallcarbonate sind beispielsweise Natriumcarbonat und Kaliumcarbonat. Geeignete Erdalkalimetallcarbonate sind beispielsweise Magnesiumcarbonat und Calciumcarbonat.

Innerhalb der Gruppe der vorgenannten Versiegelungsreagenz (b1) in Form eines Alkalisierungsmittels haben sich Ammoniak, C₂-C₆-Alkanolaminene, basischen Aminosäuren und Alkalimetallhydroxide als ganz besonders gut geeignet herausgestellt.

Im Rahmen einer weiteren besonders bevorzugten Ausführungsform ist das Verfahren dadurch gekennzeichnet, dass das Mittel (b) als Versiegelungsreagenz (b1) mindestens ein Alkalisierungsmittel ausgewählt aus der Gruppe aus Ammoniak, C₂-C₆-Alkanolaminenen, basischen Aminosäuren und Alkalimetallhydroxiden umfasst.

Im Rahmen einer weiteren besonders bevorzugten Ausführungsform ist das Verfahren dadurch gekennzeichnet, dass das Mittel (b) als Versiegelungsreagenz (b1) mindestens ein Alkalisierungsmittel enthält, das ausgewählt ist aus der Gruppe aus Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid und Kaliumhydroxid.

Das Mittel (b) enthält das Alkalisierungsmittel als Versiegelungsreagenz (b1) in einem kosmetischen Träger, bevorzugt in einem wässrigen kosmetischen Träger.

In diesem Zusammenhang hat es sich als bevorzugt herausgestellt, wenn das Mittel (b) - bezogen auf das Gesamtgewicht des Mittels (b) - 5,0 bis 99,0 Gew.-%, bevorzugt 15,0 bis 97,0 Gew.-%, weiter bevorzugt 25,0 bis 97,0 Gew.-%, nocht weiter bevorzugt 35,0 bis 97,0 Gew.-% und ganz besonders bevorzugt 45,0 bis 97,0 Gew.-% Wasser enthält.

Im Rahmen einer weiteren Ausführungsform ist das Verfahren dadurch gekennzeichnet dass das Mittel (b) - bezogen auf das Gesamtgewicht des Mittels (b) - 5,0 bis 99,0 Gew.-%, bevorzugt 15,0 bis 97,0 Gew.-%, weiter bevorzugt 25,0 bis 97,0 Gew.-%, nocht weiter bevorzugt 35,0 bis 97,0 Gew.-% und ganz besonders bevorzugt 45,0 bis 97,0 Gew.-% Wasser enthält.

Die in dem Mittel (b) enthaltenen Alkalisierungsmittel üben einen Einfluss auf den pH-Wert des Mittels (b) auf. Hierbei wurde gefunden, dass sich insbesondere bestimmte alkalische pH-Werte vorteilhaft auf die im Verfahren erzielbare Färbeleistung und die Echtheitseigenschaften der Färbungen auswirken.

Aus diesem Grund ist es bevorzugt, dass das Mittel (b), umfassend ein Alkalisierungsmittel als Versiegelungsreagenz (b1), einen pH-Wert von 7,0 bis 12,0, bevorzugt von 7,5 bis 11,5, weiter bevorzugt von 8,0 bis 11,0 und ganz besonders bevorzugt von 8,5 bis 9,5 besitzt.

Die Messung des pH-Wertes kann mit den üblichen aus dem Stand der Technik bekannten Methoden wie beispielsweise der pH-Wert Messung mittels Glaselektroden über Einstabmessketten oder aber über pH-Indikator-Papier erfolgen.

In einer weiteren ganz besonders bevorzugten Ausführungsform ist das Verfahren, dadurch gekennzeichnet, dass das Mittel (b) ein Alkalisierungsmittel als Versiegelungsreagenz (b1) enthält und einen pH-Wert von 7,0 bis 12,0, bevorzugt von 7,5 bis 11,5, weiter bevorzugt von 8,0 bis 11,0 und ganz besonders bevorzugt von 8,5 bis 9,5 besitzt.

Bei den pH-Werten im Sinne der vorliegenden Erfindung handelt es sich um pH-Werte, die bei einer Temperatur von 22°C gemessen wurden.

In einer noch weiteren alternativen Ausführungsform enthält das Versiegelungsreagenz (b1) ein Acidifizierungsmittel.

Besonders bevorzugt wird das Acidifizierungsmittel ausgewählt aus der Gruppe bestehend aus anorganischen Säuren, organischen Säuren und Mischungen daraus.

Gute Ergebnisse konnten erhalten werden, wenn das Mittel (b) als Versiegelungsreagenz (b1) mindestens eine anorganische Säure enthält. Geeignete anorganische Säuren sind zum Beispiel Phosphorsäure, Schwefelsäure und/oder Salzsäure, wobei Schwefelsäure besonders bevorzugt ist.

In einer weiteren bevorzugten Ausführungsform ist das Verfahren dadurch gekennzeichnet, dass das Mittel (b) als Versiegelungsreagenz (b1) mindestens ein Acidifizierungsmittel aus der Gruppe der anorganischen Säuren enthält, die bevorzugt ausgewählt ist aus der Gruppe bestehend aus Phosphorsäure, Schwefelsäure, Salzsäure und Mischungen daraus.

In einer weiteren, noch mehr bevorzugten Ausführungsform ist das Verfahren dadurch gekennzeichnet, dass das Mittel (b) als Versiegelungsreagenz (b1) Schwefelsäure enthält.

Ebenfalls gute Ergebnisse konnten erhalten werden, wenn das Mittel (b) als Versiegelungsreagenz (b1) mindestens eine organische Säure enthält. Die organische Säure ist vorzugsweise ausgewählt aus der Gruppe bestehend Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, Glycolsäure, Gluconsäure, Milchsäure, Maleinsäure, Ascorbinsäure, Äpfelsäure, Weinsäure, Citronensäure und Mischungen daraus.

In einer weiteren bevorzugten Ausführungsform ist das Verfahren dadurch gekennzeichnet, dass das Mittel (b) als Versiegelungsreagenz (b1) mindestens ein Acidifizierungsmittel aus der Gruppe der organischen Säuren enthält, wobei die organische Säure vorzugsweise ausgewählt ist aus der Gruppe bestehend aus Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, Glycolsäure, Gluconsäure, Milchsäure, Maleinsäure, Ascorbinsäure, Äpfelsäure, Weinsäure, Citronensäure und Mischungen daraus.

In einer weiteren, noch mehr bevorzugten Ausführungsform ist das Verfahren dadurch gekennzeichnet, dass das Mittel (b) als Versiegelungsreagenz (b1) Essigsäure enthält.

Ebenfalls geeignete Acidifizierungsmittel umfassen Methansulfonsäure und/oder 1-Hydroxyethan-1,1-diphosphonsäure.

Innerhalb der Gruppe der vorgenannten Versiegelungsreagenzien (b1) in Form eines Acidifizierungsmittels haben sich Schwefelsäure und/oder Essigsäure als ganz besonders gut geeignet herausgestellt.

Im Rahmen einer weiteren besonders bevorzugten Ausführungsform ist das Verfahren dadurch gekennzeichnet, dass das Mittel (b) als Versiegelungsreagenz (b1) mindestens ein Acidifizierungsmittel ausgewählt aus der Gruppe aus Schwefelsäure, Essigsäure und Mischungen daraus umfasst.

Das Mittel (b) enthält das Acidifizierungsmittel als Versiegelungsreagenz (b1) in einem kosmetischen Träger, bevorzugt in einem wässrigen kosmetischen Träger.

Die in dem Mittel (b) enthaltenen Acidifizierungsmittel üben einen Einfluss auf den pH-Wert des Mittels (b) auf. Hierbei wurde gefunden, dass sich auch saure pH-Werte vorteilhaft auf die im Verfahren erzielbare Färbeleistung und die Echtheitseigenschaften der Färbungen auswirken.

Aus diesem Grund ist es bevorzugt, dass das Mittel (b), umfassend ein Acidifizierungsmittel als Versiegelungsreagenz (b1), einen pH-Wert von 2,0 bis 6,5, bevorzugt von 3,0 bis 6,0, weiter bevorzugt von 4,0 bis 6,0 und ganz besonders bevorzugt von 4,5 bis 5,5 besitzt.

Die Messung des pH-Wertes kann mit den üblichen aus dem Stand der Technik bekannten Methoden wie beispielsweise der pH-Wert Messung mittels Glaselektroden über Einstabmessketten oder aber über pH-Indikator-Papier erfolgen.

In einer weiteren ganz besonders bevorzugten Ausführungsform ist das Verfahren, dadurch gekennzeichnet, dass das Mittel (b) ein Acidifizierungsmittel als Versiegelungsreagenz (b1) enthält und einen pH-Wert von 2,0 bis 6,5, bevorzugt von 3,0 bis 6,0, weiter bevorzugt von 4,0 bis 6,0 und ganz besonders bevorzugt von 4,5 bis 5,5 besitzt.

Bei den pH-Werten im Sinne der vorliegenden Erfindung handelt es sich um pH-Werte, die bei einer Temperatur von 22°C gemessen wurden.

### Weitere Inhaltsstoffe in den Mitteln (a) und (b)

Die zuvor beschriebenen Mittel (a) und (b) können ferner auch noch ein oder mehrere optionale Inhaltsstoffe enthalten. Es ist aber erfindungswesentlich, dass mindestens eines der Mittel (a) und (b) ferner mindestens eine farbgebende Verbindung aus der Gruppe der Pigmente und/oder der direktziehenden Farbstoffe enthält.

Es kann bevorzugt sein, dass das Mittel (a) neben der mindestens einen organischen Siliciumverbindung aus der Gruppe der Silane mit einem, zwei oder drei Siliciumatomen (a1) ferner mindestens eine farbgebende Verbindungen ausgewählt aus der Gruppe bestehend aus Pigmenten und/oder direktziehenden Farbstoffen enthält.

Alternativ kann bevorzugt sein, dass das Mittel (b) neben dem Versiegelungsreagenz (b1) ferner mindestens eine farbgebende Verbindungen ausgewählt aus der Gruppe bestehend aus Pigmenten und/oder direktziehenden Farbstoffen enthält.

In einer ebenfalls bevorzugten Ausführungsform des Verfahrens enthalten das Mittel (a) und das Mittel (b) jeweils ferner mindestens eine farbgebende Verbindungen ausgewählt aus der Gruppe bestehend aus Pigmenten und/oder direktziehenden Farbstoffen.

Unabhängig vom Mittel (a) und/oder (b) hat sich der Einsatz von Pigmenten in diesem Zusammenhang als ganz besonders bevorzugt herausgestellt.

In einer weiteren ganz besonders bevorzugten Ausführungsform ist ein Verfahren dadurch gekennzeichnet, dass das Mittel (a) und/oder das Mittel (b) ferner mindestens eine farbgebende Verbindung aus der Gruppe der Pigmente enthält.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Pigmente können anorganischen und/oder organischen Ursprungs sein.

In einer bevorzugten Ausführungsform ist ein Verfahren dadurch gekennzeichnet, dass das Mittel (a) und/oder das Mittel (b) ferner mindestens eine farbgebende Verbindung aus der Gruppe der anorganischen und/oder organischen Pigmente enthält.

Bevorzugte Pigmente sind ausgewählt aus synthetischen oder natürlichen anorganischen Pigmenten. Anorganische Pigmente natürlichen Ursprungs können beispielsweise aus Kreide, Ocker, Umbra, Grünerde, gebranntem Terra di Siena oder Graphit hergestellt werden. Weiterhin können als anorganische Pigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Pigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Ebenfalls besonders bevorzugte Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Entsprechend ist ein bevorzugtes Verfahren dadurch gekennzeichnet, dass das Mittel (a) und/oder das Mittel (b) ferner mindestens eine farbgebende Verbindung aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Basis von natürlichem oder synthetischem Glimmer, die mit mindestens einem Metalloxid und/oder einem Metalloxychlorid beschichtet sind.

In einer weiteren bevorzugten Ausführungsform ist das Verfahren dadurch gekennzeichnet, dass das Mittel (a) und/oder das Mittel (b) mindestens eine farbgebende Verbindung aus der Gruppe der Pigmente enthält, die ausgewählt ist aus Pigmenten auf Basis von natürlichem oder synthetischem Glimmer, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Weitere geeignete Pigmente basieren auf Metalloxid-beschichteten plättchenförmigen Borosilikaten. Diese sind beispielsweise mit Zinnoxid, Eisenoxid(en), Siliciumdioxid und/oder Titandioxid beschichtet. Solche Borosilikat-basierten Pigmente sind beispielsweise unter der Bezeichnung MIRAGE von Eckart oder Reflecks von BASF SE erhältlich.

In einer bevorzugten Ausführungsform ist Mittel (a) dadurch gekennzeichnet, dass es mindestens eine farbgebende Verbindung aus der Gruppe der anorganischen Pigmente enthält, die ausgewählt ist aus der Gruppe bestehend aus schwarzem Eisenoxid (CI 77499), gelbem Eisenoxid (CI 77492), rotem Eisenoxid (CI 77491) und Mischungen daraus.

Gelbes Eisenoxid (oder auch Eisenoxidgelb) ist die Bezeichnung für FeO(OH), im Colour Index unter C.I. Pigment Yellow 42 gelistet.

Rotes Eisenoxid (oder auch Eisenoxidrot) ist die Bezeichnung für Fe₂O₃, im Colour Index unter C.I. Pigment Red 101 gelistet. Je nach Teilchengröße können rote Eisenoxidpigmente sehr gelbstichig (kleine Teilchengröße) bis sehr blaustichig (grobe Teilchen) eingestellt werden.

Schwarzes Eisenoxid (oder auch Eisenoxidschwarz) ist im Colour Index unter C.I. Pigment Black 11 gelistet. Eisenoxidschwarz ist ferromagnetisch. Die chemische Formel wird häufig mit Fe₃O₄ angegeben, in Wirklichkeit liegt ein Mischkristall aus Fe₂O₃ und FeO mit inverser Spinellstruktur vor. Durch Dotierung mit Chrom, Kupfer oder Mangan werden weitere Schwarzpigmente erhalten. Braunes Schwarzes Eisenoxid (oder auch Eisenoxidbraun) bezeichnet meist kein definiertes Pigment, sondern eine Mischung aus gelben, roten und/oder schwarzem Eisenoxid.

Eisenoxidpigmente weisen üblicherweise Teilchendurchmessern im Bereich von 2.000 bis 4.000 nm auf. Für einige Anwendungszwecke, insbesondere für kosmetische Zwecke kann es vorteilhaft sein, Eisenoxidpigmente mit deutlichen kleineren Teilchendurchmessern einzusetzen. So zeigen Haarfärbungen mit Eisenoxidpigmenten, die einen Teilchendurchmesser im Bereich von 100 bis 1.000 nm, mehr bevorzugt 150 nm 700 nm, aufweisen, eine bessere Haltbarkeit und eine bessere Grauabdeckung.

Noch mehr bevorzugt ist deshalb ein Mittel (a), welches ferner eine farbgebende Verbindung aus der Gruppe der Pigmente und/oder direktziehenden Farbstoffe umfasst, wobei die farbgebende Verbindung ein Pigment aus der Gruppe der Eisenoxidpigmente umfasst und wobei das Eisenoxidpigment einen Teilchendurchmesser im Bereich von 100 bis 1.000 nm, mehr bevorzugt 150 nm 700 nm, aufweist.

Beispiele für besonders geeignete Pigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} oder SynCrystal von der Firma Eckart Cosmetic Colors, Flamenco^{®}, Cellini^{®}, Cloisonné^{®}, Duocrome^{®}, Gemtone^{®}, Timica^{®}, MultiReflections, Chione von der Firma BASF SE und Sunshine^{®} von der Firma Sunstar erhältlich.
Ganz besonders bevorzugte Pigmente mit der Handelsbezeichnung Colorona^{®} sind beispielsweise: Colorona Copper, Merck, MICA, CI 77491 (IRON OXIDES)
Colorona Copper Fine, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES) Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)
Colorona^{®} SynCopper, Merck, Synthetic Fluorphlogopite (and) Iron Oxides
Colorona^{®} SynBronze, Merck, Synthetic Fluorphlogopite (and) Iron Oxides
Weiterhin besonders bevorzugte Pigmente mit der Handelsbezeichnung Xirona^{®} sind beispielsweise:
Xirona^{®} Golden Sky, Merck, Silica, CI 77891 (Titanium Dioxide), Tin Oxide
Xirona^{®} Caribbean Blue, Merck, Mica, CI 77891 (Titanium Dioxide), Silica, Tin Oxide
Xirona^{®} Kiwi Rose, Merck, Silica, CI 77891 (Titanium Dioxide), Tin Oxide
Xirona^{®} Magic Mauve, Merck, Silica, CI 77891 (Titanium Dioxide), Tin Oxide
Xirona^{®} Le Rouge, Merck, Iron Oxides (and) Silica
Zudem sind besonders bevorzugte Pigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Ebenfalls besonders bevorzugte Pigmente mit der Handelsbezeichnung Flamenco^{®} sind beispielsweise:
Flamenco^{®} Summit Turquoise T30D, BASF, Titanium Dioxide (and) Mica
Flamenco^{®} Super Violet 530Z, BASF, Mica (and) Titanium Dioxide

Im Rahmen einer weiteren Ausführungsform kann das in dem Verfahren eingesetzte Mittel (a) und/oder Mittel (b) auch ein oder mehrere farbgebende Verbindungen aus der Gruppe der organischen Pigmente enthalten.

Bei den organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen-, Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist das Verfahren dadurch gekennzeichnet, dass das Mittel (a) und/oder das Mittel (b) mindestens eine farbgebende Verbindungen aus der Gruppe der organischen Pigmente enthält, die ausgewählt ist aus der Gruppe bestehend aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Im Rahmen einer weiteren Ausführungsform des Verfahrens kann das Mittel (a) und/oder das Mittel (b) auch ein oder mehrere farbgebende Verbindungen aus der Gruppe der organischen Pigmente enthalten.

In einer weiteren besonders bevorzugten Ausführungsform ist ein Verfahren dadurch gekennzeichnet, dass das Mittel (a) und/oder das Mittel (b) mindestens eine farbgebende Verbindung aus der Gruppe der organischen Pigmente enthält, das ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Ebenfalls geeignete farbgebende Verbindungen aus der Gruppe der Pigmente sind anorganische und/oder organische Pigmente, die mit einem Polymer modifiziert wurden. Durch die Polymermodifikation kann beispielsweise die Affinität der Pigmente zu dem jeweiligen Material der mindestens einen Schicht erhöht werden.

In dem Mittel (a) und/oder dem Mittel (b) können auch so genannte Metalleffektpigmente als farbgebende Verbindung eingesetzt werden.

Die Metalleffektpigmente können insbesondere Pigmente auf Basis eines lamellaren Substratplättchens, Pigmente auf Basis von lentikularen Substratplättchen und/oder Pigmente auf Basis von Substratplättchen, die "vacuum metallized pigments" (VMP) umfassen, enthalten. Bei diesen Metalleffektpigmenten umfassen die Substratplättchen ein Metall, vorzugsweise Alumnium, oder eine Legierung. Metallsubstratplättchen-basierte Metalleffektpigmente weisen vorzugsweise eine Beschichtung auf, welche unter anderem als Schutzschicht wirkt.

Geeignete Metalleffektpigmente umfassen beispielsweise die Pigmente Alegrace^{®} Marvelous, Alegrace^{©} Gorgeous oder Alegrace^{®} Aurous von Schlenk Metallic Pigments.

Ebenfalls geeignete Metalleffektpigmente sind die Aluminium-basierten Pigmente der Reihe SILVERDREAM sowie die auf Aluminium oder auf kupfer-/ zinkhaltigen Metalllegierungen basierenden Pigmente der Reihe VISIONAIRE von Eckart.

Aufgrund ihrer ausgezeichneten Licht- und Temperaturbeständigkeit ist die Verwendung der zuvor genannten Pigmente in dem Mittel (a) und/oder (b) besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1 bis 50 µm, vorzugsweise von 5 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

In einer weiteren bevorzugten Ausführungsform ist das Verfahren dadurch gekennzeichnet, dass das Mittel (a) - bezogen auf das Gesamtgewicht des Mittels (a) - ferner ein oder mehrere farbgebende Verbindung(en) in Form von Pigmenten in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

In einer weiteren, ebenfalls bevorzugten Ausführungsform ist das Verfahren dadurch gekennzeichnet, dass das Mittel (b) - bezogen auf das Gesamtgewicht des Mittels (b) - ferner ein oder mehrere farbgebende Verbindung(en) in Form von Pigmenten in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

Als farbgebende Verbindung(en) können die in dem Verfahren angewendeten Mittel (a) und/oder Mittel (b) auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1 g/L.

Direktziehende Farbstoffe können in anionische, kationische und nichtionische direktziehende Farbstoffe unterteilt werden.

In einer weiteren bevorzugten Ausführungsform ist das Verfahren dadurch gekennzeichnet, dass das Mittel (a) und/oder das Mittel (b) ferner als farbgebende Verbindung mindestens einen anionischen, kationischen und/oder nichtionischen direktziehenden Farbstoff enthält.

In einer weiteren bevorzugten Ausführungsform ist das Verfahren dadurch gekennzeichnet, dass das Mittel (a) und/oder das Mittel (b) ferner mindestens eine farbgebende Verbindung aus der Gruppe der anionischen, nichtionischen, und/oder kationischen direktziehenden Farbstoffe enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeignete nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Im Zuge der zu dieser Erfindung führenden Arbeiten hat sich herausgestellt, dass insbesondere mit Mitteln (a) und/oder (b), die mindestens einen anionischen direktziehenden Farbstoff enthalten, Färbungen mit besonders hoher Farbintensität erzeugt werden können.

In einer explizit ganz besonders bevorzugten Ausführungsform ist das Verfahren daher dadurch gekennzeichnet, dass das Mittel (a) und/oder das Mittel (b) ferner mindestens einen anionischen direktziehenden Farbstoff als farbgebende Verbindung enthält.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO-, -SO₃-vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Die Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer Ausführungsform bevorzugt ist damit ein Verfahren zum Färben von keratinischem Material, welches dadurch gekennzeichnet ist, dass das Mittel (a) und/oder das Mittel (b) ferner mindestens einen anionischen direktziehenden Farbstoff als farbgebende Verbindung enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe (-SO₃H) eine Natriumsulfonatgruppe (-SO₃Na) und/oder eine Kaliumsulfonatgruppe (-SO₃K) besitzen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intensität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriumsalze von Mono- und Disulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatriumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 Gew.-%.

Acid Red 33 ist das Dinatriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Ein ganz besonders bevorzugtes Verfahren ist daher dadurch gekennzeichnet, dass das Mittel (a) und/oder das Mittel (b) ferner mindestens eine farbgebende Verbindung aus der Gruppe der anionischen direktziehenden Farbstoffe enthält, die ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe, insbesondere die anionischen direktziehenden Farbstoffe, können je nach erwünschter Farbintensität in verschiedenen Mengen in dem Mittel (a) und/oder dem Mittel (b) eingesetzt werden. Besonders gute Ergebnisse konnten erhalten werden, wenn das Mittel (a) und/oder das Mittel (b) - jeweils bezogen auf sein Gesamtgewicht - ferner ein oder mehrere direktziehende Farbstoffe als farbgebende Verbindung in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

In einer weiteren bevorzugten Ausführungsform ist das Verfahren dadurch gekennzeichnet, dass das Mittel (a) und/oder das Mittel - bezogen auf das Gesamtgewicht des Mittels (a) und/oder des Mittels (b) - ferner ein oder mehrere direktziehende Farbstoffe als farbgebende Verbindung in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

Im folgenden werden bevorzugte Ausführungsformen des Verfahrens bezüglich der farbgebenden Verbindungen offenbart:
1. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen,
      (a2) mindestens eine farbgebende Verbindung, umfassend mindestens ein anorganisches Pigment, welches ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und Mischungen daraus,
   - Anwendung eines Mittels (b) auf dem keratinischen Material, wobei das Mittel (b) enthält:
      (b1) mindestens ein Versiegelungsreagenz.
2. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein anorganisches Pigment, welches ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und Mischungen daraus und mindestens ein Pigment ausgewählt aus der Gruppe bestehend aus Pigmenten auf Basis eines lamellaren, metallischen Substratplättchens, Pigmenten auf Basis eines lentikularen, metallischen Substratplättchens, Pigmenten auf Basis eines metallischen Substratplättchens, das ein "vacuum metallized pigment" (VMP) umfasst, und Mischungen daraus,
   - Anwendung eines Mittels (b) auf dem keratinischen Material, wobei das Mittel (b) enthält:
      (b1) mindestens ein Versiegelungsreagenz.
3. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein anorganisches Pigment, welches ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und Mischungen daraus und mindestens ein Pigment, umfassend α) ein Substratplättchen, umfassend Glimmer, und β) eine Beschichtung, umfassend mindestens eine erste Metalloxid(hydrat)schicht, umfassend TiO₂, SnO₂ und/oder Eisenoxid(e),
   - Anwendung eines Mittels (b) auf dem keratinischen Material, wobei das Mittel (b) enthält:
      (b1) mindestens ein Versiegelungsreagenz.
4. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein anorganisches Pigment, welches ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und Mischungen daraus,
   - Anwendung eines Mittels (b) auf dem keratinischen Material, wobei das Mittel (b) enthält:
      (b1) mindestens ein Versiegelungsreagenz, umfassend ein filmbildendes Polymer, und
      (b2) mindestens eine farbgebende Verbindung, umfassend mindestens ein Pigment ausgewählt aus der Gruppe bestehend aus Pigmenten auf Basis eines lamellaren, metallischen Substratplättchens, Pigmenten auf Basis eines lentikularen, metallischen Substratplättchens, Pigmenten auf Basis eines metallischen Substratplättchens, das ein "vacuum metallized pigment" (VMP) umfasst, und Mischungen daraus.
5. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein anorganisches Pigment, welches ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und Mischungen daraus,
   - Anwendung eines Mittels (b) auf dem keratinischen Material, wobei das Mittel (b) enthält:
      (b1) mindestens ein Versiegelungsreagenz, umfassend ein filmbildendes Polymer, und
      (b2) mindestens eine farbgebende Verbindung, umfassend ein Pigment, umfassend α) ein Substratplättchen, umfassend Glimmer, und β) eine Beschichtung, umfassend mindestens eine erste Metalloxid(hydrat)schicht, umfassend TiO₂, SnO₂ und/oder Eisenoxid(e).
6. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein anorganisches Pigment, welches ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und Mischungen daraus,
   - Anwendung eines Mittels (b) auf dem keratinischen Material, wobei das Mittel (b) enthält:
      (b1) mindestens ein Versiegelungsreagenz, umfassend ein filmbildendes Polymer, und
      (b2) mindestens eine farbgebende Verbindung, umfassend ein Pigment, umfassend α) ein Substratplättchen, umfassend Borosilikatglas, und β) eine Beschichtung, umfassend mindestens eine erste Metalloxid(hydrat)schicht, umfassend TiO₂, SnO₂, SiO₂, und/oder Eisenoxid(e).
7. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein organisches Pigment, das ausgewählt ist aus der Gruppe bestehend aus Carmin, Chinacridon, Phthalocyanin, Sorgho, blauen Pigmenten mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus,
   - Anwendung eines Mittels (b) auf dem keratinischen Material, wobei das Mittel (b) enthält:
      (b1) mindestens ein Versiegelungsreagenz.
8. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein organisches Pigment, das ausgewählt ist aus der Gruppe bestehend aus Carmin, Chinacridon, Phthalocyanin, Sorgho, blauen Pigmenten mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus, und mindestens ein Pigment ausgewählt aus der Gruppe bestehend aus Pigmenten auf Basis eines lamellaren, metallischen Substratplättchens, Pigmenten auf Basis eines lentikularen, metallischen Substratplättchens, Pigmenten auf Basis eines metallischen Substratplättchens, das ein "vacuum metallized pigment" (VMP) umfasst, und Mischungen daraus,
   - Anwendung eines Mittels (b) auf dem keratinischen Material, wobei das Mittel (b) enthält:
      (b1) mindestens ein Versiegelungsreagenz.
9. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein organisches Pigment, das ausgewählt ist aus der Gruppe bestehend aus Carmin, Chinacridon, Phthalocyanin, Sorgho, blauen Pigmenten mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus, und mindestens ein Pigment, umfassend α) ein Substratplättchen, umfassend Glimmer, und β) eine Beschichtung, umfassend mindestens eine erste Metalloxid(hydrat)schicht, umfassend TiO₂, SnO₂ und/oder Eisenoxid(e),
   - Anwendung eines Mittels (b) auf dem keratinischen Material, wobei das Mittel (b) enthält:
      (b1) mindestens ein Versiegelungsreagenz.
10. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein organisches Pigment, das ausgewählt ist aus der Gruppe bestehend aus Carmin, Chinacridon, Phthalocyanin, Sorgho, blauen Pigmenten mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus,
   - Anwendung eines Mittels (b) auf dem keratinischen Material, wobei das Mittel (b) enthält:
      (b1) mindestens ein Versiegelungsreagenz, umfassend ein filmbildendes Polymer, und
      (b2) mindestens eine farbgebende Verbindung, umfassend mindestens ein Pigment ausgewählt aus der Gruppe bestehend aus Pigmenten auf Basis eines lamellaren, metallischen Substratplättchens, Pigmenten auf Basis eines lentikularen, metallischen Substratplättchens, Pigmenten auf Basis eines metallischen Substratplättchens, das ein "vacuum metallized pigment" (VMP) umfasst, und Mischungen daraus.
11. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein organisches Pigment, das ausgewählt ist aus der Gruppe bestehend aus Carmin, Chinacridon, Phthalocyanin, Sorgho, blauen Pigmenten mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus,
   - Anwendung eines Mittels (b) auf dem keratinischen Material, wobei das Mittel (b) enthält:
      (b1) mindestens ein Versiegelungsreagenz, umfassend ein filmbildendes Polymer, und
      (b2) mindestens eine farbgebende Verbindung, umfassend ein Pigment, umfassend α) ein Substratplättchen, umfassend Glimmer, und β) eine Beschichtung, umfassend mindestens eine erste Metalloxid(hydrat)schicht, umfassend TiO₂, SnO₂ und/oder Eisenoxid(e).
12. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen und
      (a2) mindestens eine farbgebende Verbindung, umfassend mindestens ein organisches Pigment, das ausgewählt ist aus der Gruppe bestehend aus Carmin, Chinacridon, Phthalocyanin, Sorgho, blauen Pigmenten mit den Color Index Nummern CI 42090, CI 69800, CI 69825, CI 73000, CI 74100, CI 74160, gelben Pigmenten mit den Color Index Nummern CI 11680, CI 11710, CI 15985, CI 19140, CI 20040, CI 21100, CI 21108, CI 47000, CI 47005, grünen Pigmenten mit den Color Index Nummern CI 61565, CI 61570, CI 74260, orangen Pigmenten mit den Color Index Nummern CI 11725, CI 15510, CI 45370, CI 71105, roten Pigmenten mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus,
   - Anwendung eines Mittels (b) auf dem keratinischen Material, wobei das Mittel (b) enthält:
      (b1) mindestens ein Versiegelungsreagenz, umfassend ein filmbildendes Polymer, und
      (b2) mindestens eine farbgebende Verbindung, umfassend ein Pigment, umfassend α) ein Substratplättchen, umfassend Borosilikatglas, und β) eine Beschichtung, umfassend mindestens eine erste Metalloxid(hydrat)schicht, umfassend TiO₂, SnO₂, SiO₂, und/oder Eisenoxid(e).
13. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen,
      (a2) mindestens eine farbgebende Verbindung aus der Gruppe der Pigmente und/oder der direktziehenden Farbstoffe, umfassend ein Pigment aus der Gruppe der Eisenoxidpigmente, wobei das Eisenoxidpigment einen Teilchendurchmesser im Bereich von 100 bis 1.000 nm, mehr bevorzugt 150 nm 700 nm, aufweist,
   - Anwendung eines Mittels (b) auf dem keratinischen Material, wobei das Mittel (b) enthält:
      (b1) mindestens ein Versiegelungsreagenz.
14. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen,
      (a2) mindestens eine farbgebende Verbindung aus der Gruppe der Pigmente und/oder der direktziehenden Farbstoffe, umfassend ein Pigment aus der Gruppe der Eisenoxidpigmente, wobei das Eisenoxidpigment einen Teilchendurchmesser im Bereich von 100 bis 1.000 nm, mehr bevorzugt 150 nm 700 nm, aufweist und mindestens ein Pigment ausgewählt aus der Gruppe bestehend aus Pigmenten auf Basis eines lamellaren, metallischen Substratplättchens, Pigmenten auf Basis eines lentikularen, metallischen Substratplättchens, Pigmenten auf Basis eines metallischen Substratplättchens, das ein "vacuum metallized pigment" (VMP) umfasst, und Mischungen daraus,
   - Anwendung eines Mittels (b) auf dem keratinischen Material, wobei das Mittel (b) enthält:
      (b1) mindestens ein Versiegelungsreagenz.
15. Verfahren zum Färben von keratinischem Material, insbesondere menschlichen Haaren, umfassend die folgenden Schritte:
   - Anwendung eines Mittels (v) auf dem keratinischem Material, wobei das Mittel (v) enthält:
      (v1) mindestens ein anionisches Tensid,
   - Anwendung eines Mittels (a) auf dem keratinischem Material, wobei das Mittel (a) enthält:
      (a1) mindestens eine organische Siliciumverbindung aus der Gruppe der Silane mit einem, zwei oder drei Siliciumatomen,
      (a2) mindestens eine farbgebende Verbindung aus der Gruppe der Pigmente und/oder der direktziehenden Farbstoffe, umfassend ein Pigment aus der Gruppe der Eisenoxidpigmente, wobei das Eisenoxidpigment einen Teilchendurchmesser im Bereich von 100 bis 1.000 nm, mehr bevorzugt 150 nm 700 nm, aufweist,
   - Anwendung eines Mittels (b) auf dem keratinischen Material, wobei das Mittel (b) enthält:
      (b1) mindestens ein Versiegelungsreagenz, umfassend ein filmbildendes Polymer, und
      (b2) mindestens eine farbgebende Verbindung aus der Gruppe der Pigmente und/oder der direktziehenden Farbstoffe, umfassend mindestens ein Pigment ausgewählt aus der Gruppe bestehend aus Pigmenten auf Basis eines lamellaren, metallischen Substratplättchens, Pigmenten auf Basis eines lentikularen, metallischen Substratplättchens, Pigmenten auf Basis eines metallischen Substratplättchens, das ein "vacuum metallized pigment" (VMP) umfasst, und Mischungen daraus.

Die Mittel können zusätzlich ein oder mehrere Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ -Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ -Acylsarcosin.

Die Mittel können auch zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Die anionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Das Mittel (a) und/oder das Mittel (b) kann/können ferner ein Mattierungsmittel enthalten. Geeignete Mattierungsmittel umfassen beispielsweise (modifizierte) Stärken, Wachse, Talkum und/oder (modifizierte) Kieselsäuren. Die Menge an Mattierungsmittel liegt bevorzugt zwischen 0,1 und 10 Gew.-% bezogen auf die Gesamtmenge an Mittel (a) oder Mittel (b). Bevorzugt enthält Mittel (a) ein Mattierungsmittel.

Das Mittel (a) und/oder das Mittel (b) kann/können ferner ein Verdickungsmittel enthalten.

Bei der Anwendung der Mittel (a) und/oder (b) dürfen diese nicht zu dünnflüssig sein und vom Keratinmaterial heruntertropfen. Aus diesem Grund kann es bevorzugt sein, dass das Mittel (a) und/oder (b) ferner ein Verdickungsmittel enthält.

Im Rahmen einer Ausführungsform bevorzugt ist damit ein Verfahren zum Färben von keratinischem Material, welches dadurch gekennzeichnet ist, dass das Mittel (a) und/oder das Mittel (b) ferner ein Verdickungsmittel enthält.

Die Mittel können ferner auch noch weitere Wirk-, Hilfs- und Zusatzstoffe enthalten, wie beispielsweise Lösungsmittel, Fettbestandteile wie beispielsweise der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Strukturanten wie Glucose, Maleinsäure und Milchsäure, haarkonditionierende Verbindungen wie Phospholipide, beispielsweise Lecithin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Verfahren zum Färben von keratinischen Materialien

Im Rahmen des erfindungsgemäßen Verfahrens werden die Mittel (v), (a) und (b) auf die keratinischen Materialien, insbesondere auf die menschlichen Haare, appliziert. Damit sind die Mittel (v), (a) und (b) die anwendungsbereiten Mittel. Die Mittel (v), (a) und (b) sind voneinander verschieden.

Die Mittel (a) und (b) werden sukzessive angewendet. Das Mittel (v) wird vor den Mitteln (a) und (b) angewendet.

Die besten Ergebnisse konnten erhalten werden, wenn zunächst in einem ersten Schritt das Mittel (v) auf den keratinischen Materialien angewendet wurde, in einem zweiten Schritt das Mittel (a) auf die keratinischen Materialien gegeben wurde und in einem dritten Schritt das Mittel (b) angewendet wurde.

Um dem gefärbten keratinischen Material über einen längeren Zeitraum eine hohe Auswaschresistenz zu verleihen, werden die Mittel (v), (a) und (b) innerhalb ein und desselben Färbeverfahrens angewendet, was bedeutet, dass zwischen der Anwendung der Mittel (v) und (b) ein Zeitraum von maximal einigen Stunden liegt.

Im Rahmen einer weiteren bevorzugten Ausführungsform ist das Verfahren dadurch gekennzeichnet, das zunächst das Mittel (v), danach das Mittel (a) und danach das Mittel (b) angewendet wird, wobei der Zeitraum zwischen der Anwendung der Mittel (v) und (b) bei maximal 24 Stunden, bevorzugt bei maximal 12 Stunden und besonders bevorzugt bei maximal 6 Stunden liegt.

Ein kennzeichnendes Merkmal des Mittels (a) ist sein Gehalt an mindestens einer reaktiven organische Siliciumverbindung (a1). Das oder die reaktiven organischen Siliciumverbindungen (a1) gehen eine Oligomerisierungs- oder Polymerisierungsreaktion ein und funktionalisieren auf diesem Wege die Haaroberfläche, sobald sie mit dieser in Kontakt kommen. Im zweiten Schritt des Verfahrens wird nun ein zweites Mittel (b) auf die Haare aufgetragen. Während der Anwendung des Mittels (b), umfassend mindestens ein filmbildendes Polymer als Versiegelungsreagenz (b1), geht dieses eine Wechselwirkung mit dem Silan-Film ein und werden auf diese Weise an die keratinischen Materialien gebunden. Während der Anwendung des Mittels (b), umfassend mindestens ein Alkalisierungsmittel oder Acidifizierungsmittel als Versiegelungsreagenz (b1), wird die Ausbildung des Silan-Films positiv beeinflusst. Die gewünschte Färbung des keratinischen Materials erfolgt mit Hilfe der farbgebenden Verbindung in Mittel (a) und/oder in Mittel (b). Dabei kann die Färbung durch einen gefärbten Silan-Film (die farbgebende Verbindung ist nur in Mittel (a)), durch einen gefärbten Polymerfilm (die farbgebende Verbindung ist nur in Mittel (b) und dieses enthält ein filmbildendes Polymer als Versiegelungsreagenz (b1)) oder durch einen gefärbten Silan-Film und durch einen gefärbten Polymerfilm (Mittel (a) und (b) enthalten jeweils mindestens eine farbgebende Verbindung und Mittel (b) enthält ein filmbildendes Polymer als Versiegelungsreagenz (b1)) erhalten werden.

Im Rahmen einer weiteren Ausführungsform ist ein Verfahren ganz besonders bevorzugt, umfassend die folgenden Schritte in der angegebenen Reihenfolge
(1) Anwendung des Mittels (v) auf dem keratinischen Material,
(2) Einwirken lassen des Mittels (v) für einen Zeitraum von 10 Sekunden bis 10 Minuten, bevorzugt von 10 Sekunden bis 5 Minuten,
(3) Ausspülen des keratinischen Materials mit Wasser,
(4) Anwendung des Mittels (a) auf dem keratinischen Material,
(5) Einwirken lassen des Mittels (a) für einen Zeitraum von 10 Sekunden bis 10 Minuten, bevorzugt von 10 Sekunden bis 5 Minuten,
(6) gegebenenfalls Ausspülen des keratinischen Materials mit Wasser,
(7) Anwendung des Mittels (b) auf dem keratinischen Material,
(8) Einwirken lassen des Mittels (b) für einen Zeitraum von 30 Sekunden bis 30 Minuten, bevorzugt von 30 Sekunden bis 10 Minuten,
(9) Ausspülen des keratinischen Materials mit Wasser.

Unter dem Ausspülen des keratinischen Materials mit Wasser in den Schritten (6) und (9) des Verfahrens wird erfindungsgemäß verstanden, dass für den Ausspülvorgang nur Wasser verwendet wird, ohne dass noch weitere, von den Mitteln (a) und (b) verschiedene Mittel zur Anwendung kämen.

In einem Schritt (1) wird zunächst das Mittel (v) auf die keratinischen Materialien, insbesondere die menschlichen Haare, appliziert.

Nach dem Auftragen wird das Mittel (v) auf die keratinischen Materialien einwirken gelassen. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf den Haaren haben sich als besonders vorteilhaft erwiesen.

Im Rahmen einer bevorzugten Ausführungsform des Verfahrens wird das Mittel (v) nun von den keratinischen Materialien ausgespült werden, bevor das Mittel (a) im nachfolgenden Schritt auf die Haare appliziert wird.

In einem Schritt (4) wird das Mittel (a) auf die keratinischen Materialien, insbesondere die menschlichen Haare, appliziert.

Nach dem Auftragen wird das Mittel (a) auf die keratinischen Materialien einwirken gelassen. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf den Haaren haben sich als besonders vorteilhaft erwiesen.

Im Rahmen einer bevorzugten Ausführungsform des Verfahrens kann das Mittel (a) nun von den keratinischen Materialien ausgespült werden, bevor das Mittel (b) im nachfolgenden Schritt auf die Haare appliziert wird.

Färbungen mit ebenfalls guten Waschechtheiten wurden erhalten, wenn das Mittel (b) auf die keratinischen Materialien appliziert wurde, die noch mit dem Mittel (a) beaufschlagt waren.

In Schritt (7) wird nun das Mittel (b) auf die keratinischen Materialien appliziert. Nach dem Auftragen wird nun das Mittel (b) auf die Haare einwirken gelassen.

Das Verfahren erlaubt selbst bei kurzer Einwirkzeit des Mittels (b) die Erzeugung von Färbungen mit besonders guter Intensität und Waschechtheit. Einwirkzeiten von 10 Sekunden bis 10 Minuten, bevorzugt von 20 Sekunden bis 5 Minuten und ganz besonders bevorzugt von 30 Sekunden bis 3 Minuten auf den Haaren haben sich als besonders vorteilhaft erwiesen.

In Schritt (9) wird nun das Mittel (b) (sowie ggf. noch vorhandenes Mittel (a)) mit Wasser aus dem keratinischen Material ausgespült.

In Rahmen dieser Ausführungsform erfolgt die Abfolge der Schritte (1) bis (9) bevorzugt innerhalb von 24 Stunden.

Das Mittel (a) enthält mit der oder den organischen Siliciumverbindungen eine Klasse von hochreaktiven Verbindungen, die bei ihrer Anwendung eine Hydrolyse oder Oligomerisierung und/oder Polymerisierung eingehen können. Infolge ihrer hohen Reaktivität bilden diese organischen Siliciumverbindungen auf dem keratinischen Material einen Film aus.

Zur Vermeidung der vorzeitigen Oligomerisierung bzw. Polymerisierung ist es für den Anwender von wesentlichem Vorteil, das anwendungsbereite Mittel (a) erst kurz vor der Anwendung herstellen.

Im Rahmen einer weiteren Ausführungsform bevorzugt ist ein Verfahren, umfassend die folgenden Schritte in der angegebenen Reihenfolge
(1) Anwendung eines Mittels (v) mit einen pH-Wert im Bereich von 9,8 bis 10,2 auf dem keratinischem Material, wobei das Mittel (v) enthält:
   (a1) mindestens ein anionisches Tensid,
(2) Herstellung eines Mittels (a) durch Vermischen eines ersten Mittels (a') und eines zweiten Mittels (a"), wobei
   das erste Mittel (a') mindestens eine organische Siliciumverbindung (a1) aus der Gruppe der Silane mit einem, zwei oder drei Siliciumatomen enthält, und
   das zweite Mittel (a") Wasser und, ggf. mindestens eine farbgebende Verbindung aus der Gruppe der Pigmente und/oder direktziehenden Farbstoffe (a2) enthält,
(3) Anwendung des Mittels (a) auf dem keratinischen Material,
(4) Einwirken lassen des Mittels (a) für einen Zeitraum von 10 Sekunden bis 10 Minuten, bevorzugt von 10 Sekunden bis 5 Minuten,
(5) gegebenenfalls Ausspülen des keratinischen Materials mit Wasser,
(6) Anwendung des Mittels (b) auf dem keratinischen Material,
(7) Einwirken lassen des Mittels (b) für einen Zeitraum von 30 Sekunden bis 30 Minuten, bevorzugt von 30 Sekunden bis 10 Minuten,
(8) Ausspülen des keratinischen Materials mit Wasser.

Um eine möglichst lagerstabile Formulierung bereitstellen zu können, wird das Mittel (a') selbst bevorzugt wasserarm oder wasserfrei konfektioniert.

In einer bevorzugten Ausführungsform ist ein Verfahren dadurch gekennzeichnet, dass das Mittel (a') - bezogen auf das Gesamtgewicht des Mittels (a') - einen Wassergehalt von 0,001 bis 10 Gew.-%, bevorzugt von 0,5 bis 9 Gew.-%, weiter bevorzugt von 1 bis 8 Gew.-% und ganz besonders bevorzugt von 1,5 bis 7 Gew.-% enthält.

Das Mittel (a") kann ferner ein Verdickungsmittel enthalten.

In einer bevorzugten Ausführungsform ist ein Verfahren dadurch gekennzeichnet, dass das Mittel (a‴) - bezogen auf das Gesamtgewicht des Mittels (a") - einen Wassergehalt von 15 bis 99,9 Gew.-%, bevorzugt von 35 bis 99 Gew.-%, weiter bevorzugt von 55 bis 99 Gew.-%, noch weiter bevorzugt von 65 bis 99 Gew.-% und ganz besonders bevorzugt von 75 bis 99 Gew.-% besitzt.

Innerhalb dieser Ausführungsform wird das anwendungsbereite Mittel (a) durch Vermischen der Mittel (a') und (a") hergestellt.

Beispielsweise kann der Anwender das Mittel (a'), welches die organische Siliciumverbindung(en) (a1) enthält, zunächst mit dem Wasser-haltigen Mittel (a") verrühren oder verschütteln. Diese Mischung aus (a') und (a") kann der Anwender nun - entweder direkt nach ihrer Herstellung oder nach einer kurzen Reaktionszeit von 10 Sekunden bis 20 Minuten - auf die keratinischen Materialien applizieren. Im Anschluss daran kann der Anwender wie zuvor beschrieben das Mittel (b) anwenden.

Es kann auch bevorzugt sein, wenn in dem Verfahren ferner ein Mittel (a‴) eingesetzt wird, welches mindestens eine farbgebende Verbindung aus der Gruppe der Pigmente und/oder direktziehenden Farbstoffe (a2) enthält.

Im Rahmen einer weiteren Ausführungsform besonders bevorzugt ist ein Verfahren, umfassend die folgenden Schritte in der angegebenen Reihenfolge
(1) Anwendung eines Mittels (v) mit einen pH-Wert im Bereich von 9,8 bis 10,2 auf dem keratinischem Material, wobei das Mittel (v) enthält:
   (a1) mindestens ein anionisches Tensid,
(2) Einwirken lassen des Mittels (a) für einen Zeitraum von 10 Sekunden bis 10 Minuten, bevorzugt von 10 Sekunden bis 5 Minuten,
(3) Ausspülen des keratinischen Materials mit Wasser,
(4) Herstellung eines Mittels (a) durch Vermischen eines ersten Mittels (a'), eines zweiten Mittels (a") und eines dritten Mittels (a‴), wobei
   - das erste Mittel (a') mindestens eine organische Siliciumverbindung (a1) aus der Gruppe der Silane mit einem, zwei oder drei Siliciumatomen enthält,
   - das zweite Mittel (a") Wasser, und
   - das dritte Mittel (a‴) mindestens eine farbgebende Verbindung aus der Gruppe der Pigmente und/oder direktziehenden Farbstoffe (a2) enthält
(5) Anwendung des Mittels (a) auf dem keratinischen Material,
(6) Einwirken lassen des Mittels (a) für einen Zeitraum von 10 Sekunden bis 10 Minuten, bevorzugt von 10 Sekunden bis 5 Minuten,
(7) gegebenenfalls Ausspülen des keratinischen Materials mit Wasser,
(8) Anwendung des Mittels (b) auf dem keratinischen Material,
(9) Einwirken lassen des Mittels (b) für einen Zeitraum von 30 Sekunden bis 30 Minuten, bevorzugt von 30 Sekunden bis 10 Minuten,
(10) Ausspülen des keratinischen Materials mit Wasser.

Innerhalb dieser Ausführungsform wird das anwendungsbereite Mittel (a) durch Vermischen der Mittel (a'), (a") und (a‴) hergestellt.

Beispielsweise kann der Anwender das Mittel (a'), welches die organische Siliciumverbindung(en) (a1) enthält, zunächst mit dem Wasser-haltigen Mittel (a") und anschließend mit dem die mindestens eine farbgebende Verbindung enthaltenden Mittel (a‴) verrühren oder verschütteln. Diese Mischung aus (a'), (a") und (a‴) kann der Anwender nun - entweder direkt nach ihrer Herstellung oder nach einer kurzen Reaktionszeit von 10 Sekunden bis 20 Minuten - auf die keratinischen Materialien applizieren. Im Anschluss daran kann der Anwender wie zuvor beschrieben das Mittel (b) anwenden. Beispielsweise kann der Anwender das Mittel (a‴), das die mindestens eine farbgebende Verbindung enthält, zunächst mit dem Wasser-haltigen Mittel (a") und anschließend mit dem Mittel (a'), welches die organische Siliciumverbindung(en) (a1) enthält, verrühren oder verschütteln. Diese Mischung aus (a'), (a") und (a‴) kann der Anwender nun - entweder direkt nach ihrer Herstellung oder nach einer kurzen Reaktionszeit von 10 Sekunden bis 20 Minuten - auf die keratinischen Materialien applizieren. Im Anschluss daran kann der Anwender wie zuvor beschrieben das Mittel (b) anwenden.

Auch das anwendungsbereite Mittel (b) kann, insbesondere wenn es mindestens eine farbgebende Verbindung enthält, erst kurz vor der Anwendung des Mittels (b) hergestellt werden. In diesem Fall wird das anwendungsbereite Mittel (b) beispielsweise durch Vermischen der Mittel (b') und (b") hergestellt.

Beispielsweise kann der Anwender das Mittel (b'), welches das Versiegelungsreagenz (b1) enthält, und das Mittel (b"), das die mindestens eine farbgebende Verbindung enthält, verrühren oder verschütteln. Diese Mischung aus (b') und (b") kann der Anwender nun - entweder direkt nach ihrer Herstellung oder nach einer kurzen Reaktionszeit von 10 Sekunden bis 20 Minuten - auf die keratinischen Materialien applizieren.

Die mindestens eine farbgebende Verbindung (a2) und/oder (b2) aus der Gruppe der Pigmente und der direktziehenden Farbstoffe wird vorzugsweise in Form einer Pigmentsuspension, umfassend die mindestens eine farbgebende Verbindung (a2) und/oder (b2) und ein flüssiges Trägermedium, eingesetzt. Das Trägermedium ist vorzugsweise nicht-wässrig. Das Trägermedium kann beispielsweise ein Silikonöl umfassen. Entsprechend können die Mittel (a) und (b) neben den beiden jeweils zwingend enthaltenen Inhaltsstoffen (a1) und (b1) sowie der mindestens einen farbgebenden Verbindung (a2) und/oder (b2) ferner jeweils das flüssige Trägermedium umfassen.

### Mehrkomponenten-Verpackungseinheit (Kit-of-parts)

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Ein zweiter Gegenstand der vorliegenden Erfindung ist daher eine Mehrkomponenten-Verpackungseinheit (Kit-of-parts) zum Färben von keratinischem Material, umfassend getrennt voneinander konfektioniert
- einen ersten Container mit einem Mittel (v) mit einen pH-Wert im Bereich von 9,8 bis 10,2, wobei das Mittel (v) enthält:
   (v1) mindestens ein anionisches Tensid,
- einen zweiten Container mit einem Mittel (a'), wobei das Mittel (a') enthält:
   (a1) mindestens eine organische Siliciumverbindung aus der Gruppe der Silane mit einem, zwei oder drei Siliciumatomen, und
- einen dritten Container mit einem Mittel (a"), wobei das Mittel (a") enthält:
   Wasser, und
- einen vierten Container mit einem Mittel (b), wobei das Mittel (b) enthält:
   (b1) mindestens ein Versiegelungsreagenz, umfassend eine Verbindung ausgewählt aus der Gruppe bestehend aus filmbildenden Polymeren, Alkalisierungsmitteln, Acidifizierungsmitteln und Mischungen daraus,
wobei die Bestandteile (a1) und (b1) im Detail oben offenbart wurden und mindestens eines der Mittel (a") und (b) ferner mindestens eine farbgebende Verbindung aus der Gruppe der Pigmente und/oder der direktziehenden Farbstoffe enthält.

Eine bevorzugte Ausführungsform umfasst eine Mehrkomponenten-Verpackungseinheit (Kit-of-parts) zum Färben von keratinischem Material, umfassend getrennt voneinander konfektioniert
- einen ersten Container mit einem Mittel (v) mit einen pH-Wert im Bereich von 9,8 bis 10,2, wobei das Mittel (v) enthält:
   (v1) mindestens ein anionisches Tensid,
- einen zweiten Container mit einem Mittel (a'), wobei das Mittel (a') enthält:
   (a1) mindestens eine organische Siliciumverbindung aus der Gruppe der Silane mit einem, zwei oder drei Siliciumatomen, und
- einen dritten Container mit einem Mittel (a"), wobei das Mittel (a") enthält:
   Wasser, und
- einen vierten Container mit einem Mittel (a‴), wobei das Mittel (a‴) enthält:
   (a2) mindestens eine farbgebende Verbindung aus der Gruppe der Pigmente und/oder der direktziehenden Farbstoffe
- einen fünften Container mit einem Mittel (b), wobei das Mittel (b) enthält:
- (b1) mindestens ein Versiegelungsreagenz, umfassend eine Verbindung ausgewählt aus der Gruppe bestehend aus filmbildenden Polymeren, Alkalisierungsmitteln, Acidifizierungsmitteln und Mischungen daraus,
wobei die Bestandteile (v1), (a1), (a2) und (b1) im Detail oben offenbart wurden.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (v) mit einen pH-Wert im Bereich von 9,8 bis 10,2, wobei das Mittel (v) enthält:
   (v1) mindestens ein anionisches Tensid,
- einen zweiten Container mit einem Mittel (a'), wobei das Mittel (a') enthält:
   mindestens eine organische Siliciumverbindung (a1) aus der Gruppe der Silane mit einem, zwei oder drei Siliciumatomen, und
- einen dritten Container mit einem Mittel (a"), wobei das Mittel (a") enthält:
   Wasser und
   (a2) mindestens eine farbgebende Verbindung aus der Gruppe der Pigmente und/oder der direktziehenden Farbstoffe, und
- einen vierten Container mit einem Mittel (b), wobei das Mittel (b) enthält:
   (b1) mindestens ein Versiegelungsreagenz, umfassend eine Verbindung ausgewählt aus der Gruppe bestehend aus filmbildenden Polymeren, Alkalisierungsmitteln, Acidifizierungsmitteln und Mischungen daraus,
wobei die Bestandteile (v1), (a1), (a2) und (b1) im Detail oben offenbart wurden.

Im Rahmen noch einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (v) mit einen pH-Wert im Bereich von 9,8 bis 10,2, wobei das Mittel (v) enthält:
   (v1) mindestens ein anionisches Tensid,
- einen zweiten Container mit einem Mittel (a'), wobei das Mittel (a') enthält:
   mindestens eine organische Siliciumverbindung (a1) aus der Gruppe der Silane mit einem, zwei oder drei Siliciumatomen, und
- einen dritten Container mit einem Mittel (a"), wobei das Mittel (a") enthält:
   (a2) mindestens eine farbgebende Verbindung aus der Gruppe der Pigmente und/oder der direktziehenden Farbstoffe, und
- einen vierten Container mit einem Mittel (b), wobei das Mittel (b) enthält:
   (b1) mindestens ein Versiegelungsreagenz, umfassend eine Verbindung ausgewählt aus der Gruppe bestehend aus filmbildenden Polymeren, Alkalisierungsmitteln, Acidifizierungsmitteln und Mischungen daraus,
wobei die Bestandteile (v1), (a1), (a2) und (b1) im Detail oben offenbart wurden.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (v) mit einen pH-Wert im Bereich von 9,8 bis 10,2, wobei das Mittel (v) enthält:
   (v1) mindestens ein anionisches Tensid,
- einen zweiten Container mit einem Mittel (a'), wobei das Mittel (a') enthält:
   mindestens eine organische Siliciumverbindung (a1) aus der Gruppe der Silane mit einem, zwei oder drei Siliciumatomen, und
- einen dritten Container mit einem Mittel (a"), wobei das Mittel (a") enthält:
   Wasser, und
- einen vierten Container mit einem Mittel (b), wobei das Mittel (b) enthält:
   (b1) mindestens ein Versiegelungsreagenz, umfassend ein filmbildendes Polymer, und ferner
   (b2) eine farbgebende Verbindung aus der Gruppe der Pigmente und/oder der direktziehenden Farbstoffe,
wobei die Bestandteile (v1), (a1), (b1) und (b2) im Detail oben offenbart wurden.

Im Rahmen noch einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (v) mit einen pH-Wert im Bereich von 9,8 bis 10,2, wobei das Mittel (v) enthält:
   (v1) mindestens ein anionisches Tensid,
- einen zweiten Container mit einem Mittel (a'), wobei das Mittel (a') enthält:
   mindestens eine organische Siliciumverbindung (a1) aus der Gruppe der Silane mit einem, zwei oder drei Siliciumatomen, und
- einen dritten Container mit einem Mittel (a"), wobei das Mittel (a") enthält:
   Wasser
- einen vierten Container mit einem Mittel (a‴), wobei das Mittel (a‴) enthält:
   (a2) mindestens eine farbgebende Verbindung aus der Gruppe der Pigmente und/oder der direktziehenden Farbstoffe,
- einen fünften Container mit einem Mittel (b'), wobei das Mittel (b') enthält:
   (b1) mindestens ein Versiegelungsreagenz, umfassend ein filmbildendes Polymer, und
- einen sechsten Container mit einem Mittel (b"), wobei das Mittel (b") enthält:
   (b2) mindestens eine farbgebende Verbindung aus der Gruppe der Pigmente und/oder der direktziehenden Farbstoffe,
wobei die Bestandteile (v1), (a1), (a2), (b1) und (b2) im Detail oben offenbart wurden.

Im Rahmen noch einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (v) mit einen pH-Wert im Bereich von 9,8 bis 10,2, wobei das Mittel (v) enthält:
   (v1) mindestens ein anionisches Tensid,
- einen zweiten Container mit einem Mittel (a'), wobei das Mittel (a') enthält:
   mindestens eine organische Siliciumverbindung (a1) aus der Gruppe der Silane mit einem, zwei oder drei Siliciumatomen, und
- einen dritten Container mit einem Mittel (a"), wobei das Mittel (a") enthält:
   Wasser
- einen vierten Container mit einem Mittel (b'), wobei das Mittel (b') enthält:
   (b1) mindestens ein Versiegelungsreagenz, umfassend ein filmbildendes Polymer, und
- einen fünften Container mit einem Mittel (b"), wobei das Mittel (b") enthält:
   (b2) mindestens eine farbgebende Verbindung aus der Gruppe der Pigmente und/oder der direktziehenden Farbstoffe,
wobei die Bestandteile (v1), (a1), (b1) und (b2) im Detail oben offenbart wurden.

Betreffend die weiteren bevorzugten Ausführungsformen der Mehrkomponenten-Verpackungseinheiten gilt *mutatis mutandis* das zum Verfahren Gesagte.

### Beispiele

Es wurden die folgenden Formulierungen hergestellt (sofern nichts anderes angegeben ist, sind alle Angaben in Gew.-% Aktivsubstanz)

### Mittel (v)

| Mittel (v) | Gew.-% | | |
|---|---|---|---|
| | (vI) | (vII) | (vIII) |
| Sodium Lauryl Sulfate (INCI) (v1) | 6,8 | 3,5 | -- |
| Sodium Laureth Sulfate (INCI) (v1) | -- | 6 | 12 |
| Wasser | ad 100 | | |

Der pH-Wert der Vorbehandlungsmittel (vI) bis (vIII) wurde mit Hilfe von NaOH auf einen pH-Wert von 10 eingestellt.

### Mittel (a')

| Mittel (a') | Gew.-% |
|---|---|
| (3-Aminopropyl)triethoxysilan (a1) | 24 |
| Methyltriethoxysilan (a1) | 72 |
| Wasser | ad 100 |

### Mittel (a")

| Mittel (a") | Gew.-% |
|---|---|
| Wasser | 100 |

### Mittel (a‴)

| Mittel (a‴) | Gew.-% |
|---|---|
| Pigmentmischung (CI 12490, CI 74160 und CI 11680) | 1 |
| PEG-12 Dimethicone | ad 100 |

Das anwendungsbereite Mittel (a) wurde durch Vermischen von 5 g des Mittels (a'), 5 g des Mittels (a‴) und 15 g Wasser (Mittel (a")) hergestellt. Der pH-Wert des Mittels (a) wurde durch Zugabe von Ammoniak bzw. Milchsäure auf einen Wert von 10,5 eingestellt. Dann wurde das Mittel (a) für ca. 5 Minuten stehen gelassen.

### Mittel (b)

| Mittel (b) | Gew.-% |
|---|---|
| Ethylene/Sodium Acrylate Copolymer (b1) (25%ige Lösung) | 40 |
| Timiron SynWhite Satin (ex Merck) | 5 |
| Wasser | ad 100 |

Das Mittel (a) wurde jeweils in eine Haarsträhne (Kerling, Euronaturhaar weiß) einmassiert, und für 1 Minute einwirken gelassen. Danach wurde das Mittel (a) mit Wasser ausgespült.

Im Anschluss daran wurde das Mittel (b) auf die Haarsträhne appliziert, für 1 Minute einwirken gelassen und danach ebenfalls mit Wasser ausgespült.

## Patentansprüche

1. Verfahren zum Färben von keratinischem Material, insbesondere menschlichen Haaren, umfassend die folgenden Schritte:
- in einem ersten Schritt Anwendung eines Mittels (v) mit einen pH-Wert im Bereich von 9,8 bis 10,2 auf dem keratinischem Material, wobei das Mittel (v) enthält:
(v1) mindestens ein anionisches Tensid,
- in einem zweiten Schritt Anwendung eines Mittels (a) auf dem keratinischem Material, wobei das Mittel (a) enthält:
(a1) mindestens eine organische Siliciumverbindung aus der Gruppe der Silane mit einem, zwei oder drei Siliciumatomen und
- in einem dritten Schritt Anwendung eines Mittels (b) auf dem keratinischen Material, wobei das Mittel (b) enthält:
(b1) mindestens ein Versiegelungsreagenz, umfassend eine Verbindung ausgewählt aus der Gruppe bestehend aus filmbildenden Polymeren, Alkalisierungsmitteln, Acidifizierungsmitteln und Mischungen daraus,
wobei mindestens eines der Mittel (a) und (b) ferner mindestens eine farbgebende Verbindung aus der Gruppe der Pigmente und/oder der direktziehenden Farbstoffe enthält.

2. Verfahren nach Anspruch 1, **dadurch gekennzeichnet, dass** das Mittel (a) mindestens eine organische Siliciumverbindung (a1) der Formel (I) und/oder (II) enthält
R₁R₂N-L-Si(OR₃)ₐ(R₄)_{b} (I),
wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃, R₄ unabhängig voneinander für eine C₁-C₆-Alkylgruppe stehen,
- a, für eine ganze Zahl von 1 bis 3 steht, und
- b für die ganze Zahl 3 - a steht, und
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} und R_{6"} unabhängig voneinander für eine C₁-C₆-Alkylgruppe stehen,
- A, A', A", A‴ und Aʺʺ unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkylgruppe oder eine Gruppierung der Formel (III) stehen
- (Aʺʺ)-Si(R₆")_{d}"(OR₅")_{c}" (III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Verfahren nach einem der Ansprüche 1 bis 2, **dadurch gekennzeichnet, dass** das Mittel (a) mindestens eine organische Siliciumverbindung (a1) der Formel (I) enthält,
R₁R₂N-L-Si(OR₃)ₐ(R₄)_{b} (I),
wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht.

4. Verfahren nach einem der Ansprüche 1 bis 3, **dadurch gekennzeichnet, dass** das Mittel (a) mindestens eine organische Siliciumverbindung (a1) der Formel (I) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan und/oder
- 1-(2-Dimethylaminoethyl)silantriol.

5. Verfahren nach einem der Ansprüche 1 bis 4, **dadurch gekennzeichnet, dass** das Mittel (a) mindestens eine organische Siliciumverbindung (a1) der Formel (II) enthält,
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

6. Verfahren nach einem der Ansprüche 1 bis 5, **dadurch gekennzeichnet, dass** das Mittel (a) mindestens eine organische Siliciumverbindung (a1) der Formel (IV) enthält.
R₉Si(OR₁₀)ₖ(R₁₁)ₘ (IV),
wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

7. Verfahren nach einem der Ansprüche 1 bis 6, **dadurch gekennzeichnet, dass** das Mittel (a) mindestens eine organische Siliciumverbindung (a1) der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
- Octadecyltrimethoxysilan,
- Octadecyltriethoxysilan und
- Mischungen daraus.

8. Verfahren nach einem der Ansprüche 1 bis 7, **dadurch gekennzeichnet, dass** das Mittel (a) mindestens zwei strukturell voneinander verschiedene organische Siliciumverbindungen (a1) enthält.

9. Verfahren nach einem der Ansprüche 1 bis 8, **dadurch gekennzeichnet, dass** das Mittel (v) mindestens ein anionisches Tensid (v1) ausgewählt aus der Gruppe bestehend aus:
- geradkettigen oder verzweigten, gesättigten oder ein- oder mehrfach ungesättigten Alkylsulfonaten mit 8 bis 24 C-Atomen,
- linearen Alpha-Olefinsulfonaten mit 8 bis 24 C-Atomen,
- Alkylsulfaten und Alkylpolyglykolethersulfaten der Formel R₉-O-(CH₂-CH₂O)ₙ-SO₃X, in der R₉ bevorzugt für einen geradkettigen oder verzweigten, gesättigten oder ein- oder mehrfach ungesättigten Alkyl- oder Alkenlyrest mit 8 bis 24 Kohlenstoffatomen, n für 0 oder 1 bis 12, und X für ein Alkali- oder Erdalkalimetallion oder für protoniertes Triethanolamin oder das Ammonium-Ion steht,
- geradkettigen oder verzweigten, gesättigten oder ein- oder mehrfach ungesättigten Alkylcarbonsäuren mit 8 bis 24 C-Atomen,
- geradkettigen oder verzweigten, gesättigten oder ein- oder mehrfach ungesättigten Alkylphosphaten mit 8 bis 24 C-Atomen,
- Acylisethionaten, deren Acylgruppe ausgewählt ist aus einer verzweigten oder unverzweigten C₆ bis C₂₂ Alkylgruppe
- Alkylglycosidcarbonsäuren, dessen Alkylgruppe ausgewählt ist aus einer verzweigten oder unverzweigten C₆ bis C₂₂ Alkylgruppe,
- Alkylsulfosuccinaten, deren zwei Alkylgruppen ausgewählt sind aus gleichen oder verschiedenen, verzweigte oder unverzweigten C₂ bis C₁₂ Alkylgruppen,
- N-Acyltauraten, deren Acylgruppe ausgewählt ist aus einer verzweigten oder unverzweigten C₆ bis C₂₂ Alkylgruppe,
- Alkylsarcosinaten, deren Alkylgruppe ausgewählt ist aus einer verzweigten oder unverzweigten C₆ bis C₂₂ Alkylgruppe,
- Sulfonate ungesättigter Fettsäuren mit 8 bis 24 C-Atomen und 1 bis 6 Doppelbindungen und
- Mischungen daraus,
wobei das/die Gegenion(en) des anionischen Tensids Alkali- oder Erdalkalimetallionen, protoniertes Triethanolamin oder Ammonium-Ionen umfasst/umfassen.

10. Verfahren nach einem der Ansprüche 1 bis 9, **dadurch gekennzeichnet, dass** das Mittel (v) ein anionisches Tensid aus der Gruppe der C₈₋₁₈-Alkylsulfatsalze, insbesondere das Natriumsalz von Laurylsulfat (INCI: Sodium Lauryl Sulfate), umfasst..

11. Verfahren nach einem der Ansprüche 1 bis 10, **dadurch gekennzeichnet, dass** das Mittel (v) mindestens zwei anionische Tenside (v1) enthält.

12. Verfahren nach einem der Ansprüche 1 bis 11, **dadurch gekennzeichnet, dass** das Mittel (v) mindestens zwei anionische Tenside (v1) enthält, umfassend eine Verbindung mit der INCI-Bezeichnung Sodium Lauryl Sulfate und/oder eine Verbindung mit der INCI-Bezeichnung Sodium Laureth Sulfate.

13. Mehrkomponenten-Verpackungseinheit (Kit-of-parts) zum Färben von keratinischem Material, umfassend getrennt voneinander konfektioniert
- einen ersten Container mit einem Mittel (v) mit einen pH-Wert im Bereich von 9,8 bis 10,2, wobei das Mittel (v) enthält:
(v1) mindestens ein anionisches Tensid,
- einen zweiten Container mit einem Mittel (a'), wobei das Mittel (a') enthält:
(a1) mindestens eine organische Siliciumverbindung aus der Gruppe der Silane mit einem, zwei oder drei Siliciumatomen, und
- einen dritten Container mit einem Mittel (a"), wobei das Mittel (a") enthält:
Wasser, und
- einen vierten Container mit einem Mittel (a‴), wobei das Mittel (a‴) enthält:
(a2) mindestens eine farbgebende Verbindung aus der Gruppe der Pigmente und/oder der direktziehenden Farbstoffe
- einen fünften Container mit einem Mittel (b), wobei das Mittel (b) enthält:
(b1) mindestens ein Versiegelungsreagenz, umfassend eine Verbindung ausgewählt aus der Gruppe bestehend aus filmbildenden Polymeren, Alkalisierungsmitteln, Acidifizierungsmitteln und Mischungen daraus.

## Claims

1. A method for dyeing keratinous material, in particular human hair, comprising the following steps:
- in a first step, applying a composition (v) having a pH in the range of 9.8 to 10.2 to the keratinous material, wherein the composition (v) contains:
(v1) at least one anionic surfactant,
- in a second step, applying a composition (a) to the keratinous material, wherein the composition (a) contains:
(a1) at least one organic silicon compound from the group of silanes having one, two, or three silicon atoms, and
- in a third step, applying a composition (b) to the keratinous material, wherein the composition (b) contains:
(b1) at least one sealing recomposition comprising a compound selected from the group consisting of film-forming polymers, alkalizing compositions, acidifying compositions, and mixtures thereof,
wherein at least one of the compositions (a) and (b) further contains at least one coloring compound from the group of pigments and/or direct dyes.

2. The method according to claim 1, **characterized in that** composition (a) contains at least one organic silicon compound (a1) of formula (I) and/or (II)
R₁R₂N-L-Si(OR₃)ₐ(R₄)_{b} (1),
wherein
- R₁ and R₂ independently represent a hydrogen atom or a C₁-C₆ alkyl group,
- L represents a linear or branched, divalent C₁-C₂₀ alkylene group,
- R₃ and R₄ independently represent a C₁-C₆ alkyl group,
- a represents an integer from 1 to 3, and
- b represents the integer 3 - a, and
wherein, in the organosilicon compound of formula (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'}, and R_{6"} independently represent a C₁-C₆ alkyl group,
- A, A', A", A‴, and Aʺʺ independently represent a linear or branched, divalent C₁-C₂₀ alkylene group,
- R₇ and R₈ independently represent a hydrogen atom, a C₁-C₆ alkyl group, a hydroxy-C₁-C₆ alkyl group, a C₂-C₆ alkenyl group, an amino-C₁-C₆ alkyl group, or a group of formula (III)
- (Aʺʺ)-Si(R₆")_{d}"(OR₅")_{c}" (III),
- c represents an integer from 1 to 3,
- d represents the integer 3 - c,
- c' represents an integer from 1 to 3,
- d' represents the integer 3 - c',
- c" represents an integer from 1 to 3,
- d" represents the integer 3 - c",
- e represents 0 or 1,
- f represents 0 or 1,
- g represents 0 or 1,
- h represents 0 or 1,
provided that at least one of the radicals e, f, g, and h is nonzero.

3. A method according to any one of claims 1 through 2, **characterized in that** the composition (a) contains at least one organic silicon compound (a1) of formula (I),
R₁R₂N-L-Si(OR₃)ₐ(R₄)_{b} (I),
wherein
- R₁ and R₂ both represent a hydrogen atom, and
- L represents a linear, divalent C₁-C₆ alkylene group, preferably a propylene group (-CH₂-CH₂-CH₂-) or an ethylene group (-CH₂-CH₂-),
- R₃ and R₄ independently represent a methyl group or an ethyl group,
- a represents the number 3, and
- b represents the number 0.

4. A method according to any one of claims 1 through 3, **characterized in that** the composition (a) contains at least one organic silicon compound (a1) of formula (I), which is selected from the group consisting of
- (3-aminopropyl)triethoxysilane
- (3-aminopropyl)trimethoxysilane
- 1-(3-aminopropyl)silanetriol
- (2-aminoethyl)triethoxysilane
- (2-aminoethyl)trimethoxysilane
- 1-(2-aminoethyl)silanetriol
- (3-dimethylaminopropyl)triethoxysilane
- (3-dimethylaminopropyl)trimethoxysilane
- 1-(3-dimethylaminopropyl)silane triol
- (2-dimethylaminoethyl)triethoxysilane.
- (2-dimethylaminoethyl)trimethoxysilane and/or
- 1-(2-dimethylaminoethyl)silantriol.

5. A method according to any one of claims 1 through 4, **characterized in that** the composition (a) contains at least one organic silicon compound (a1) of formula (II),
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
where
- e and f both represent the number 1,
- g and h both represent the number 0,
- A and A' independently represent a linear, divalent C₁-C₆-alkylene group, and
- R7 represents a hydrogen atom, a methyl group, a 2-hydroxyethyl group, a 2-alkenyl group, a 2-aminoethyl group, or a group of formula (III).

6. A method according to any one of claims 1 through 5, **characterized in that** the composition (a) contains at least one organic silicon compound (a1) of formula (IV).
R₉Si(OR₁₀)ₖ(R₁₁)ₘ (IV),
wherein
- R₉ represents a C₁-C₁₈ alkyl group,
- R₁₀ represents a hydrogen atom or a C₁-C₆ alkyl group,
- R₁₁ represents a C₁-C₆ alkyl group
- k represents an integer from 1 to 3, and
- m represents the integer 3 - k.

7. A method according to any one of claims 1 through 6, **characterized in that** the composition (a) contains at least one organic silicon compound (a1) of formula (IV), selected from the group consisting of
- methyltrimethoxysilane
- methyltriethoxysilane
- ethyltrimethoxysilane
- ethyltriethoxysilane
- propyltrimethoxysilane
- propyltriethoxysilane
- hexyltrimethoxysilane
- hexyltriethoxysilane
- octyltrimethoxysilane
- octyltriethoxysilane
- dodecyltrimethoxysilane,
- dodecyltriethoxysilane,
- octadecyltrimethoxysilane,
- octadecyltriethoxysilane, and
- mixtures thereof.

8. A method according to any one of claims 1 through 7, **characterized in that** the composition (a) contains at least two structurally distinct organic silicon compounds (a1).

9. A method according to any one of claims 1 through 8, **characterized in that** the composition (v) contains at least one anionic surfactant (v1) selected from the group consisting of:
- straight-chain or branched, saturated or mono- or polyunsaturated alkyl sulfonates having 8 to 24 carbon atoms,
- linear alpha-olefin sulfonates having 8 to 24 carbon atoms,
- alkyl sulfates and alkyl polyglycol ether sulfates of the formula R₉-O-(CH₂-CH₂O)ₙ-SO₃X, in which R₉ preferably represents a straight-chain or branched, saturated or mono- or polyunsaturated alkyl or alkenyl radical having 8 to 24 carbon atoms, n represents 0 or 1 through 12, and X represents an alkali or alkaline earth metal ion, protonated triethanolamine, or the ammonium ion,
- straight-chain or branched, saturated or mono- or polyunsaturated alkyl carboxylic acids having 8 to 24 carbon atoms,
- straight-chain or branched, saturated or mono- or polyunsaturated alkyl phosphates having 8 to 24 carbon atoms,
- acyl isethionates, wherein the acyl group is selected from a branched or unbranched C₆ to C₂₂ alkyl group
- alkyl glycoside carboxylic acids, whose alkyl group is selected from a branched or unbranched C₆ to C₂₂ alkyl group,
- alkyl sulfosuccinates, whose two alkyl groups are selected from identical or different, branched or unbranched C₂ to C₁₂ alkyl groups,
- N-acyltaurates, whose acyl group is selected from a branched or unbranched C₆ to C₂₂ alkyl group,
- alkylsarcosinates, whose alkyl group is selected from a branched or unbranched C₆ to C₂₂ alkyl group,
- sulfonates of unsaturated fatty acids having 8 to 24 carbon atoms and 1 to 6 double bonds, and
- mixtures thereof,
wherein the counterion(s) of the anionic surfactant comprise alkali or alkaline earth metal ions, protonated triethanolamine, or ammonium ions.

10. A method according to any one of claims 1 through 9, **characterized in that** the composition (v) comprises an anionic surfactant from the group of C₈₋₁₈ alkyl sulfate salts, in particular the sodium salt of lauryl sulfate (INCI: Sodium Lauryl Sulfate).

11. A method according to any one of claims 1 through 10, **characterized in that** the composition (v) contains at least two anionic surfactants (v1).

12. A method according to any one of claims 1 through 11, **characterized in that** the composition (v) contains at least two anionic surfactants (v1), comprising a compound with the INCI designation Sodium Lauryl Sulfate and/or a compound with the INCI designation Sodium Laureth Sulfate.

13. A multi-component packaging unit (kit-of-parts) for coloring keratinous material, comprising the following, packaged separately from one another
- a first container containing a composition (v) with a pH value in the range of 9.8 to 10.2, wherein the composition (v) contains:
(v1) at least one anionic surfactant,
- a second container containing a composition (a'), wherein the composition (a') contains:
(a1) at least one organic silicon compound from the group of silanes having one, two, or three silicon atoms, and
- a third container containing a composition (a"), wherein the composition (a") comprises:
water, and
- a fourth container containing a composition (aʺʺ), wherein the composition (aʺʺ) comprises:
(a2) at least one coloring compound selected from the group consisting of pigments and/or direct dyes
- a fifth container containing an composition (b), wherein the composition (b) contains:
(b1) at least one sealing recomposition comprising a compound selected from the group consisting of film-forming polymers, alkalizing compositions, acidifying compositions, and mixtures thereof.

## Revendications

1. Procédé de coloration d'une matière kératinique, en particulier des cheveux humains, comprenant les étapes suivantes:
- dans une première étape, l'application sur la matière kératinique d'un agent (v) dont le pH est compris entre 9,8 et 10,2, ledit agent (v) contenant:
(v1) au moins un tensioactif anionique,
- dans une deuxième étape, l'application d'un agent (a) sur la matière kératinique, ledit agent (a) contenant:
(a1) au moins un composé organique du silicium appartenant au groupe des silanes comportant un, deux ou trois atomes de silicium et
- dans une troisième étape, l'application d'un agent (b) sur la matière kératinique, ledit agent (b) contenant:
(b1) au moins un agent de scellement comprenant un composé choisi dans le groupe constitué par les polymères filmogènes, les agents alcalinisants, les agents acidifiants et leurs mélanges,
au moins l'un des agents (a) et (b) contenant en outre au moins un composé colorant appartenant au groupe des pigments et/ou des colorants directs.

2. Procédé selon la revendication 1, **caractérisé en ce que** l'agent (a) contient au moins un composé organique du silicium (a1) de formule (1) et/ou (II)
R₁R₂N-L-Si(OR₃)ₐ(R₄)_{b} (1),
où
- R₁, R₂ représentent, indépendamment l'un de l'autre, un atome d'hydrogène ou un groupe alkyle en C₁-C₆,
- L représente un groupe alkylène en C₁-C₂₀ divalent, linéaire ou ramifié,
- R₃ et R₄ représentent, indépendamment l'un de l'autre, un groupe alkyle en C₁ à C₆,
- a représente un nombre entier compris entre 1 et 3, et
- b représente le nombre entier 3 - a, et
dans lequel, dans le composé organique du silicium de formule (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} et R_{6"} représentent, indépendamment les uns des autres, un groupe alkyle en C₁ à C₆,
- A, A', A", A‴ et Aʺʺ représentent, indépendamment les uns des autres, un groupe alkylène divalent linéaire ou ramifié en C₁ à C₂₀,
- R₇ et R₈ représentent, indépendamment l'un de l'autre, un atome d'hydrogène, un groupe alkyle en C₁ à C₆, un groupe hydroxyalkyle en C₁ à C₆, un groupe alcényle en C₂ à C₆, un groupe aminoalkyle en C₁ à C₆ ou un groupement de formule (III)
- (A‴‴)-Si(R₆ʺʺ)_{d}ʺʺ(OR₅ʺʺ)_{c}ʺʺ (III),
- c représente un nombre entier compris entre 1 et 3,
- d représente le nombre entier 3 - c,
- c' représente un nombre entier compris entre 1 et 3,
- d' représente le nombre entier 3 - c',
- c" représente un nombre entier compris entre 1 et 3,
- d«« représente le nombre entier 3 - c«« ;
- e vaut 0 ou 1 ;
- f vaut 0 ou 1 ;
- g vaut 0 ou 1 ;
- h vaut 0 ou 1 ;
à condition qu'au moins l'un des radicaux parmi e, f, g et h soit différent de 0.

3. Procédé selon l'une des revendications 1 à 2, **caractérisé en ce que** l'agent (a) contient au moins un composé organique du silicium (a1) de formule (I),
R₁R₂N-L-Si(OR₃)ₐ(R₄)_{b} (I),
dans laquelle
- R₁ et R₂ représentent tous deux un atome d'hydrogène, et
- L représente un groupe alkylène linéaire bivalent en C₁ à C₆, de préférence un groupe propylène (-CH₂-CH₂-CH₂-) ou un groupe éthylène (-CH₂-CH₂-),
- R₃ et R₄ représentent, indépendamment l'un de l'autre, un groupe méthyle ou un groupe éthyle,
- a représente le nombre 3 et
- b représente le nombre 0.

4. Procédé selon l'une des revendications 1 à 3, **caractérisé en ce que** l'agent (a) contient au moins un composé organique du silicium (a1) de formule (1), choisi dans le groupe constitué par
- le (3-aminopropyl)triéthoxysilane
- le (3-aminopropyl)triméthoxysilane
- 1-(3-aminopropyl)silantriol
- (2-aminoéthyl)triéthoxysilane
- (2-aminoéthyl)triméthoxysilane
- 1-(2-aminoéthyl)silantriol
- (3-diméthylaminopropyl)triéthoxysilane
- (3-diméthylaminopropyl)triméthoxysilane
- 1-(3-diméthylaminopropyl)silane-triol
- (2-diméthylaminoéthyl)triéthoxysilane.
- (2-diméthylaminoéthyl)triméthoxysilane et/ou
- 1-(2-diméthylaminoéthyl)silantriol.

5. Procédé selon l'une des revendications 1 à 4, **caractérisé en ce que** l'agent (a) contient au moins un composé organique du silicium (a1) de formule (II),
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
où
- e et f représentent tous deux le nombre 1,
- g et h représentent tous deux le nombre 0,
- A et A' représentent, indépendamment l'un de l'autre, un groupe alkylène linéaire bivalent en C₁ à C₆, et
- R7 représente un atome d'hydrogène, un groupe méthyle, un groupe 2-hydroxyéthyle, un groupe 2-alcényle, un groupe 2-aminoéthyle ou un groupement de formule (III).

6. Procédé selon l'une des revendications 1 à 5, **caractérisé en ce que** l'agent (a) contient au moins un composé organique du silicium (a1) de formule (IV).
R₉Si(OR₁₀)ₖ(R₁₁)ₘ (IV),
où
- R₉ représente un groupe alkyle en C₁ à C₁₈,
- R₁₀ représente un atome d'hydrogène ou un groupe alkyle en C₁ à C₆,
- R₁₁ représente un groupe alkyle en C₁ à C₆
- k représente un nombre entier compris entre 1 et 3, et
- m représente le nombre entier 3 - k.

7. Procédé selon l'une des revendications 1 à 6, **caractérisé en ce que** l'agent (a) contient au moins un composé organique du silicium (a1) de formule (IV), choisi dans le groupe constitué par
- le méthyltriméthoxysilane
- le méthyltriéthoxysilane
- l'éthyltriméthoxysilane
- éthyltriéthoxysilane
- propyltriméthoxysilane
- propyltriéthoxysilane
- hexyltriméthoxysilane
- hexyltriéthoxysilane
- octyltriméthoxysilane
- octyltriéthoxysilane
- dodécyltriméthoxysilane,
- dodécyltriéthoxysilane,
- octadécyltriméthoxysilane,
- octadécyltriéthoxysilane et
- leurs mélanges.

8. Procédé selon l'une des revendications 1 à 7, **caractérisé en ce que** l'agent (a) contient au moins deux composés organiques du silicium (a1) de structures différentes l'une de l'autre.

9. Procédé selon l'une des revendications 1 à 8, **caractérisé en ce que** l'agent (v) contient au moins un tensioactif anionique (v1) choisi dans le groupe constitué par :
- des alkylsulfonates à chaîne linéaire ou ramifiée, saturés ou mono- ou polyinsaturés, comportant de 8 à 24 atomes de carbone,
- des alpha-oléfinesulfonates linéaires comportant de 8 à 24 atomes de carbone,
- des alkylsulfates et des alkylpolyglycoléthersulfates de formule R₉-O-(CH₂-CH₂O)ₙ-SO₃X, dans laquelle R₉ représente de préférence un radical alkyle ou alcényle à chaîne linéaire ou ramifiée, saturé ou mono- ou polyinsaturé, comportant de 8 à 24 atomes de carbone, n vaut 0 ou 1 à 12, et X représente un ion de métal alcalin ou alcalino-terreux, ou la triéthanolamine protonée, ou l'ion ammonium,
- des acides alkylcarboxyliques à chaîne linéaire ou ramifiée, saturés ou mono- ou polyinsaturés, comportant de 8 à 24 atomes de carbone,
- des alkylphosphates à chaîne linéaire ou ramifiée, saturés ou mono- ou polyinsaturés, comportant de 8 à 24 atomes de carbone,
- des acyliséthionates dont le groupe acyle est choisi parmi un groupe alkyle ramifié ou non ramifié en C₆ à C₂₂,
- des acides alkylglycosidecarboxyliques, dont le groupe alkyle est choisi parmi un groupe alkyle ramifié ou linéaire en C₆ à C₂₂,
- des alkylsulfosuccinates, dont les deux groupes alkyle sont choisis parmi des groupes alkyle identiques ou différents, ramifiés ou linéaires, en C₂ à C₁₂,
- des N-acyltaurates dont le groupe acyle est choisi parmi un groupe alkyle en C₆ à C₂₂, ramifié ou linéaire,
- des alkylsarcosinates dont le groupe alkyle est choisi parmi un groupe alkyle en C₆ à C₂₂, ramifié ou linéaire,
- des sulfonates d'acides gras insaturés comportant de 8 à 24 atomes de carbone et de 1 à 6 doubles liaisons, et
- des mélanges de ceux-ci,
le ou les contre-ions de l'agent tensioactif anionique comprenant des ions de métaux alcalins ou alcalino-terreux, de la triéthanolamine protonée ou des ions ammonium.

10. Procédé selon l'une des revendications 1 à 9, **caractérisé en ce que** l'agent (v) comprend un tensioactif anionique appartenant au groupe des sels d'alkylsulfate en C₈₋₁₈, en particulier le sel de sodium du laurylsulfate (INCI : Sodium Lauryl Sulfate).

11. Procédé selon l'une des revendications 1 à 10, **caractérisé en ce que** l'agent (v) contient au moins deux tensioactifs anioniques (v1).

12. Procédé selon l'une des revendications 1 à 11, **caractérisé en ce que** l'agent (v) contient au moins deux tensioactifs anioniques (v1), comprenant un composé portant la désignation INCI « Sodium Lauryl Sulfate » et/ou un composé portant la désignation INCI « Sodium Laureth Sulfate ».

13. Ensemble d'emballage à plusieurs composants (kit-of-parts) destiné à la coloration de matière kératinique, comprenant, conditionnés séparément les uns des autres
- un premier récipient contenant un produit (v) dont le pH est compris entre 9,8 et 10,2, ledit produit (v) contenant :
(v1) au moins un tensioactif anionique,
- un deuxième récipient contenant un agent (a'), ledit agent (a') contenant :
(a1) au moins un composé organique du silicium appartenant au groupe des silanes comportant un, deux ou trois atomes de silicium, et
- un troisième récipient contenant un agent (a‴), ledit agent (a‴) contenant :
de l'eau, et
- un quatrième récipient contenant un agent (a‴‴), ledit agent (a‴‴) contenant :
(a2) au moins un composé colorant appartenant au groupe des pigments et/ou des colorants directs
- un cinquième récipient contenant un agent (b), ledit agent (b) contenant :
(b1) au moins un agent de scellement comprenant un composé choisi dans le groupe constitué par les polymères filmogènes, les agents alcalinisants, les agents acidifiants et leurs mélanges.
